# EUROPEAN PATENT APPLICATION

(11) **EP 3 878 463 A1**
(43) Date of publication of application: **15.09.2021**
(21) Application number: 19878345.8
(22) Date of filing: 01.11.2019
(51) Int. Cl.: A61K 39/00, A61K 39/39, A61P 35/00, A61P 37/04, A61K 9/107

(54) **EMULSION PREPARATION AND PREPARATION METHOD THEREOF**

(30) Priority: 02.11.2018 JP 2018207779
(71) Applicant: Taiho Pharmaceutical Co., Ltd., Chiyoda-ku Tokyo 101-8444 (JP)
(72) Inventor: DOI Yusuke, Tokushima-shi, Tokushima 771-0194 (JP)
(74) Representative: Altmann Stößel Dick Patentanwälte PartG mbB
(86) International application number: PCT/JP2019/043023
(87) International publication number: WO 2020/091040

(57) **Abstract**

This invention provides a water-in-oil (w/o) emulsion formulation that stably contains 3 types of peptides having 4 linked CTL epitopes. This invention also provides a method for efficiently preparing the w/o emulsion formulation containing 3 types of peptides having 4 linked CTL epitopes. This invention provides a w/o emulsion formulation that stably contains 3 types of peptides having 4 linked CTL epitopes and a method for efficiently preparing such w/o emulsion formulation. In addition, this invention provides an improved method and an apparatus to prepare such emulsion formulation.

## Description

### Technical Field

The present invention relates to an emulsion formulation of peptides and a method for preparing such preparation. More specifically, the present invention relates to a water-in-oil (w/o) emulsion formulation containing CTL epitope peptides and an oil adjuvant and a method for preparing such preparation.

### [Background Art]

Cancer treatment using peptide vaccines that has drawn attention in recent years exerts effects in a manner such that T cell receptors (TCRs) of epitope-specific cytotoxic T lymphocytes (CTLs) recognize the major histocompatibility complex (MHC) on the surfaces of cancer cells presenting the antigen peptides administered and the CTLs damage cancer cells. Human MHC is referred to as "human leukocyte antigen (HLA)" and HLA types are known to be extremely diverse.

Effective cancer peptides vary depending on the HLA types of humans to which peptides are to be administered. Accordingly, HLA types targeted by a peptide vaccine for cancer are restricted. A peptide having 4 linked CTL epitopes comprising various types of CTL epitope peptides capable of CTL induction on one or more HLA types selected from among HLA-A2, HLA-A24, HLA-A26, and HLA-A3 supertypes has been reported (Patent Literature 1).

Meanwhile, oil adjuvants are known to enhance the effect of peptide vaccines for cancer. When formulating peptides especially in the form of peptide vaccines for cancer using an oil adjuvant, it is a common practice to prepare a water-in-oil (w/o) emulsion formulation. Since an emulsion formulation may develop problems such as separation or aggregation over time, it is preferred to prepare the emulsion at the time of use. Thus, a method and an apparatus used for preparing such emulsion has been proposed (Patent Literature 2 and Patent Literature 3).

### [Prior Art Literatures]

### [Patent Literatures]

[Patent Literature 1] WO 2015/060235
[Patent Literature 2] WO 2007/083763
[Patent Literature 3] JP Patent No. 5,629,882

### [Summary of the Invention]

### [Objects to Be Attained by the Invention]

A method for preparing an emulsion formulation of the peptide having 4 linked CTL epitopes as disclosed in WO 2015/060235 is disclosed in the publication thereof. However, the disclosure concerns preparation of an emulsion formulation of a single type of peptide having 4 linked CTL epitopes, and WO 2015/060235 does not disclose any emulsion formulation containing 3 types of peptides having 4 linked CTL epitopes according to the present invention. Therefore, the first object of the present invention is to provide a w/o emulsion formulation that stably contains 3 types of peptides having 4 linked CTL epitopes.

When preparing the emulsion formulation containing 3 types of peptides having 4 linked CTL epitopes according to the present invention in accordance with a standard method of emulsion preparation, the procedure for preparation is time-consuming, preparation may end up in a failure, and workers are seriously burdened in clinical situations such as hospitals where emulsion formulations are to be prepared at the time of use, although such problems were not known. The present inventors prepared the emulsion formulation containing 3 types of peptides having 4 linked CTL epitopes according to the present invention in accordance with a standard method of emulsion preparation and found these problems. Accordingly, the second object of the present invention is to provide a method for efficiently preparing the w/o emulsion formulation described above.

### [Means for Attaining the Objects]

The present inventor has conducted concentrated studies in order to attain the above objects. As a result, the inventor has discovered that an emulsion formulation could be efficiently prepared within a short period of time by mixing an aqueous phase containing peptides having 4 linked CTL epitopes with an oil phase containing an oil adjuvant in a step-wise manner. Thus, the inventor has succeeded in preparing a w/o emulsion formulation stably containing 3 types of peptides having 4 linked CTL epitopes, which has led to the completion of the present invention.

The present invention provides [1] to [13] below.
[1] A water-in-oil (w/o) emulsion formulation containing 3 types of peptides having 4 linked CTL epitopes each having 4 CTL epitope peptides via linkers in an aqueous phase.
[2] The water-in-oil emulsion formulation according to [1], which contains 3 types of peptides having 4 linked CTL epitopes and an oil adjuvant.
[3] The water-in-oil emulsion formulation according to [1] or [2], which is obtained by mixing an aqueous phase comprising peptides having 4 linked CTL epitopes and an oil phase comprising an oil adjuvant and emulsifying the mixture, wherein the aqueous phase comprising peptides having 4 linked CTL epitopes is a solution comprising 3 types of peptides having 4 linked CTL epitopes: a peptide represented by Formula (I); a peptide represented by Formula (II); and a peptide represented by Formula (III):
   A-(L)-B-(L)-C-(L)-PEP2 (I);
   D-(L)-E-(L)-F-(L)-PEP4 (II); and
   G-(L)-H-(L)-I-(L)-PEP10 (III),
   in Formulae (I), (II), and (III),
   (L) represents a linker;
   PEP2 represents a CTL epitope peptide represented by SEQ ID NO: 2;
   PEP4 represents a CTL epitope peptide represented by SEQ ID NO: 4;
   PEP10 represents a CTL epitope peptide represented by SEQ ID NO: 10;
   A, B, C, D, E, F, G, H, and I are each independently selected from the group consisting of a CTL epitope peptide represented by SEQ ID NO: 1 (PEP1), a CTL epitope peptide represented by SEQ ID NO: 5 (PEP5), a CTL epitope peptide represented by SEQ ID NO: 6 (PEP6), a CTL epitope peptide represented by SEQ ID NO: 7 (PEP7), a CTL epitope peptide represented by SEQ ID NO: 8 (PEP8), a CTL epitope peptide represented by SEQ ID NO: 9 (PEP9), a CTL epitope peptide represented by SEQ ID NO: 13 (PEP13), a CTL epitope peptide represented by SEQ ID NO: 15 (PEP15), and a CTL epitope peptide represented by SEQ ID NO: 18 (PEP18) (except for a combination of A representing PEP7 and B representing PEP8); and
   the peptides having 4 linked CTL epitopes represented by Formulae (I), (II), and (III) may each have a peptide sequence consisting of hydrophilic amino acids.
[4] The emulsion formulation according to [3], wherein, in Formulae (I), (II), and (III), (L) represents a linker; A, B, and C are each independently selected from the group consisting of PEP7, PEP8, and PEP13 (except for a combination of A representing PEP7 and B representing PEP8); D, E, and F are each independently selected from the group consisting of PEP5, PEP6, and PEP9; and G, H, and I are each independently selected from the group consisting of PEP1, PEP15, and PEP18, and the peptide represented by Formula (I) may have a peptide sequence consisting of hydrophilic amino acids.
[5] The emulsion formulation according to [3] or [4], wherein the peptide represented by Formula (I) is selected from the group consisting of:
   ▪ PEP7-(L)-PEP13 -(L)-PEP8-(L)-PEP2;
   ▪ PEP8-(L)-PEP7-(L)-PEP13-(L)-PEP2;
   ▪ PEP8-(L)-PEP13 -(L)-PEP7-(L)-PEP2;
   ▪ PEP13-(L)-PEP7-(L)-PEP8-(L)-PEP2; and
   ▪ PEP13 -(L)-PEP8-(L)-PEP7-(L)-PEP2,
   the peptide represented by Formula (II) is selected from the group consisting of:
   ▪ PEP5-(L)-PEP6-(L)-PEP9-(L)-PEP4;
   ▪ PEP5-(L)-PEP9-(L)-PEP6-(L)-PEP4;
   ▪ PEP6-(L)-PEP5-(L)-PEP9-(L)-PEP4;
   ▪ PEP6-(L)-PEP9-(L)-PEP5-(L)-PEP4;
   ▪ PEP9-(L)-PEP5-(L)-PEP6-(L)-PEP4; and
   ▪ PEP9-(L)-PEP6-(L)-PEP5-(L)-PEP4, and
   the peptide represented by Formula (III) is selected from the group consisting of:
   ▪ PEP1-(L)-PEP15-(L)-PEP18-(L)-PEP10;
   ▪ PEP1-(L)-PEP18-(L)-PEP15-(L)-PEP10;
   ▪ PEP1-5-(L)-PEP1-(L)-PEP18-(L)-PEP10;
   ▪ PEP1-5-(L)-PEP18-(L)-PEP1-(L)-PEP10;
   ▪ PEP1-8-(L)-PEP1-(L)-PEP15-(L)-PEP10; and
   ▪ PEP18-(L)-PEP15-(L)-PEP1-(L)-PEP10, and
   the peptide represented by Formula (I) may have a peptide sequence consisting of hydrophilic amino acids at the N terminus.
[6] The emulsion formulation according to any of [3] to [5], wherein, in Formulae (I), (II), and (III), (L) represents an arginine dimer; and a peptide sequence consisting of hydrophilic amino acids is an arginine tetramer.
[7] The emulsion formulation according to any of [3] to [6], wherein the 3 types of peptides having 4 linked CTL epitopes represented by Formulae (I), (II), and (III) are RRRR-PEP7-RR-PEP13-RR-PEP8-RR-PEP2 (SEQ ID NO: 19), PEP5-RR-PEP9-RR-PEP6-RR-PEP4 (SEQ ID NO: 25), and PEP15-RR-PEP18-RR-PEP1-RR-PEP10 (SEQ ID NO: 33).
[8] A method for preparing the emulsion formulation according to any of [1] to [7] comprising the steps (i) and (ii):
   (i) a step of injecting a part of an aqueous phase comprising peptides having 4 linked CTL epitopes into an oil phase comprising an oil adjuvant to prepare a mixture; and
   (ii) a step of allowing the mixture to reciprocate between syringes connected via a syringe connector,
   wherein the step (i) and the step (ii) are repeated to obtain an emulsion formulation comprising the total amount of the oil phase and the aqueous phase.
[9] The method according to [8], wherein the amount of a part of the aqueous phase is 1/10 to 1/2 fold (v/v) relative to the amount of the oil phase comprising an oil adjuvant.
[10] An apparatus used for preparing a water-in-oil emulsion formulation containing peptides, which is composed of:
   a second syringe 2;
   a syringe connector 5 connected to the second syringe 2;
   a three-way stopcock 4 connected to the syringe connector 5; and
   a first syringe 1 and a third syringe 3 further connected to the three-way stopcock 4,
   wherein the apparatus enables production of a water-in-oil emulsion formulation by adding an aqueous phase filled in the third syringe 3 to the oil phase filled in the first syringe 1 or the second syringe 2 and allowing the resulting mixture to reciprocate between the first syringe 1 and the second syringe 2 via the syringe connector 5.
[11] The apparatus according to [10], wherein the volumes of the first, the second, and the third syringes are each 3 ml or less.
[12] The apparatus according to [10] or [11], wherein an inner diameter of the syringe connector 5 through which the mixture passes is within a range from 0.8 to 1.7 mm.
[13] A kit used for preparing an w/o emulsion containing peptides having 4 linked CTL epitopes, which comprises peptides having 4 linked CTL epitopes each comprising 4 CTL epitope peptides linked via linkers, an oil adjuvant, and the apparatus according to any of [10] to [12].

The present invention also relates to the following aspects.
[14] A method for treatment of tumor of a patient comprising administering the water-in-oil (w/o) emulsion formulation according to any of [1] to [7] to the patient.
[15] The water-in-oil (w/o) emulsion formulation according to any of [1] to [7] for use in the treatment of tumor.
[16] Use of a solution of peptides having 4 linked CTL epitopes in the manufacture of the water-in-oil (w/o) emulsion formulation according to any of [1] to [7].
[17] The apparatus according to any of [10] to [12] or the kit according to [13], which is used for preparing the water-in-oil (w/o) emulsion formulation for the treatment of tumor at the time of use.
[18] The apparatus according to any of [10] to [12] or the kit according to [13], wherein the water-in-oil (w/o) emulsion formulation is the formulation according to any of [1] to [7].
[19] A method for treatment of tumor of a patient comprising:
   (a) preparing the emulsion formulation according to any of [1] to [7] by performing the step (i) and the step (ii):
      (i) a step of injecting a part of an aqueous phase comprising peptides having 4 linked CTL epitopes into an oil phase comprising an oil adjuvant to prepare a mixture; and
      (ii) a step of allowing the mixture to reciprocate between syringes connected via a syringe connector, and
      repeating the step (i) and the step (ii) to obtain the emulsion formulation comprising the total amount of the oil phase and the aqueous phase; and
   (b) administering the resulting emulsion formulation to a patient having tumor.

This description includes part or all of the content as disclosed in the description and/or drawings of Japanese Patent Application No. 2018-207779, which is a priority document of the present application.

### Effects of the Invention

According to the method of the present invention, peptides having 4 linked CTL epitopes are mixed with an oil adjuvant in a step-wise manner, so that a stable w/o emulsion formulation can be efficiently prepared within a short period of time.

### [Brief Description of the Drawings]

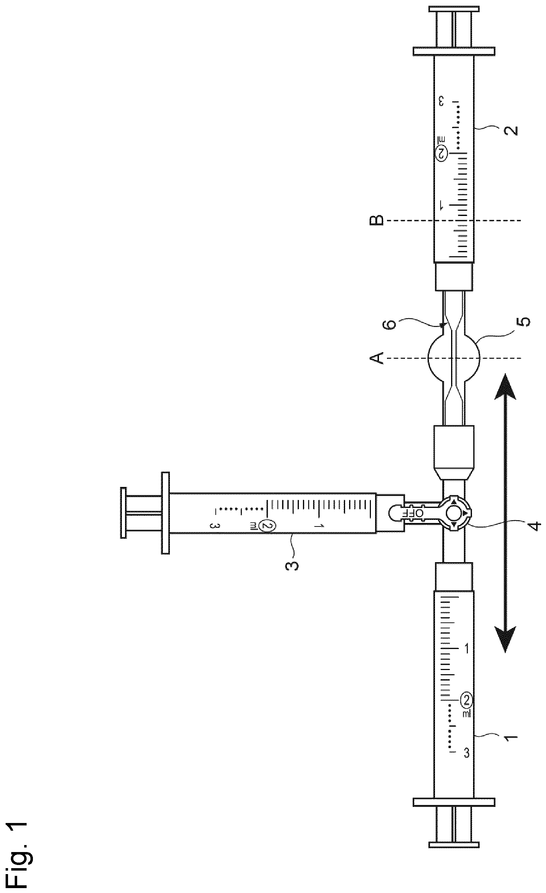

Fig. 1 schematically shows an apparatus that can be suitably used for preparing the emulsion formulation according to the present invention in which liquid can migrate among the first syringe 1, the syringe connector 5, and the second syringe 2, but can not migrate to or from the third syringe 3. An arrow indicates the direction of liquid migration.

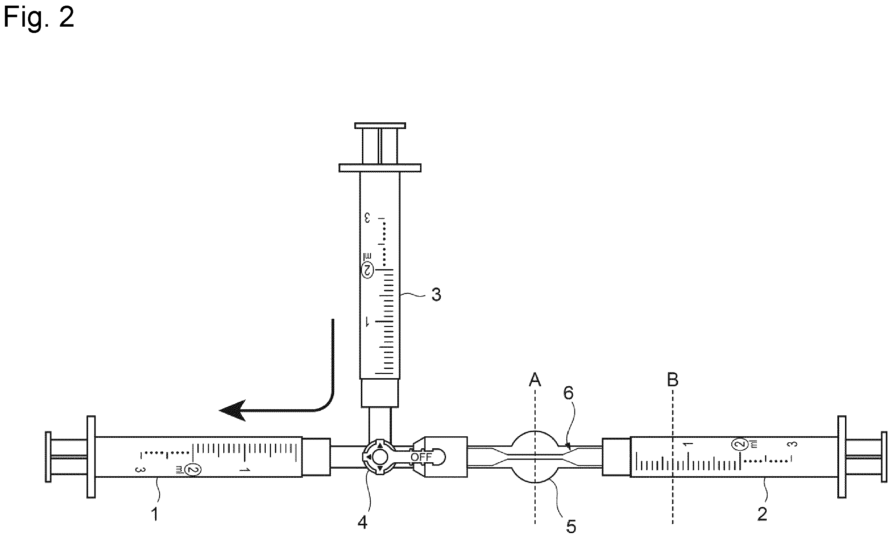

Fig. 2 schematically shows an apparatus that can be suitably used for preparing the emulsion formulation according to the present invention in which liquid can migrate between the first syringe 1 and the third syringe 3, but can not migrate to or from the second syringe 2 and the syringe connector 5. An arrow indicates the direction of liquid migration.

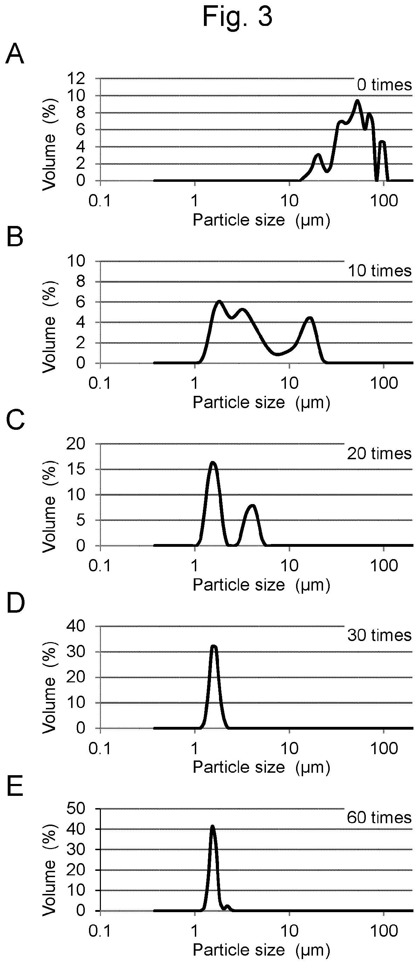

Fig. 3 shows the correlation between the number of reciprocation between syringes at the time of main emulsification when an emulsion formulation is prepared by a method of divisional preparation (A: 0 times; B: 10 times; C: 20 times: D: 30 times; E: 60 times) and particle sizes of the resulting emulsions.

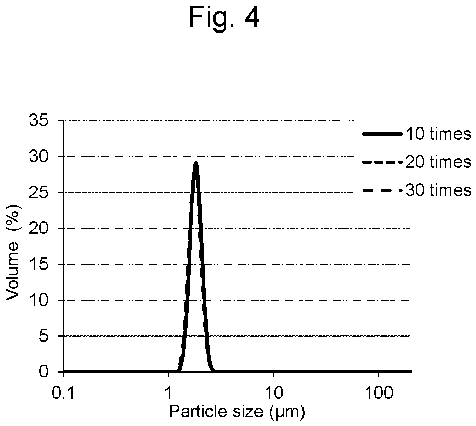

Fig. 4 shows the correlation between the number of reciprocation between syringes at the time of main emulsification when an emulsion formulation is prepared by a method of emulsion preparation using a three-way stopcock and particle sizes of the resulting emulsions.

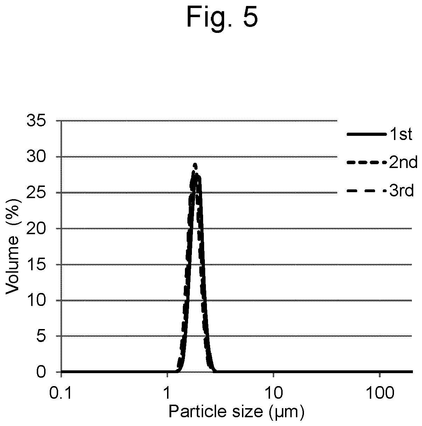

Fig. 5 shows particle size uniformity of the w/o emulsion formulation obtained by the method of the present invention.

### [Embodiments for Carrying out the Invention]

### <Water-in-oil (w/o) emulsion formulation>

The present invention provides a water-in-oil (w/o) emulsion formulation comprising 3 types of peptides having 4 linked CTL epitopes each comprising 4 CTL epitope peptides linked via linkers in an aqueous phase. More specifically, the present invention provides a w/o emulsion formulation comprising 3 types of peptides having 4 linked CTL epitopes each comprising 4 CTL epitope peptides linked via linkers and an oil adjuvant.

In the present invention, the "peptide having 4 linked CTL epitopes" means a peptide as a single molecule comprising 4 peptides each selected from among CTL epitope peptides derived from the same and/or different tumor antigen molecules linearly linked via linkers.

In the present invention, the emulsion formulation can be obtained by mixing a solution of peptides having 4 linked CTL epitopes with an oil adjuvant and emulsifying the resulting mixture. The emulsion formulation according to the present invention can be used as a stable w/o emulsion formulation.

Examples of known CTL epitope peptides derived from tumor antigen molecules include the following:
KLVERLGAA (SEQ ID NO: 1; referred to as "PEP1" herein, e.g., WO 2001/011044);
ASLDSDPWV (SEQ ID NO: 2; referred to as "PEP2" herein, e.g., WO 2002/010369);
ALVEFEDVL (SEQ ID NO: 3; referred to as "PEP3" herein, e.g., WO 2002/010369);
LLQAEAPRL (SEQ ID NO: 4; referred to as "PEP4" herein, e.g., WO 2000/12701);
DYSARWNEI (SEQ ID NO: 5; referred to as "PEP5" herein, e.g., JP H11-318455 A);
VYDYNCHVDL (SEQ ID NO: 6; referred to as "PEP6" herein, e.g., WO 2000/12701);
LYAWEPSFL (SEQ ID NO: 7; referred to as "PEP7 " herein, e.g., JP 2003-000270 A);
DYLRSVLEDF (SEQ ID NO: 8; referred to as "PEP8" herein, e.g., WO 2001/011044);
QIRPIFSNR (SEQ ID NO: 9; referred to as "PEP9" herein, e.g., WO 2008/007711);
ILEQSGEWWK (SEQ ID NO: 10; referred to as "PEP10" herein, e.g., WO 2009/022652);
VIQNLERGYR (SEQ ID NO: 11; referred to as "PEP11" herein, e.g., WO 2009/022652);
KLKHYGPGWV (SEQ ID NO: 12; referred to as "PEP12" herein, e.g., WO 1999/067288);
RLQEWCSVI (SEQ ID NO: 13; referred to as "PEP13" herein, e.g., WO 2002/010369);
ILGELREKV (SEQ ID NO: 14; referred to as "PEP14" herein, e.g., WO 2002/010369);
DYVREHKDNI (SEQ ID NO: 15; referred to as "PEP15" herein, e.g., WO 2005/071075);
HYTNASDGL (SEQ ID NO: 16; referred to as "PEP16" herein, e.g., WO 2001/011044);
NYSVRYRPGL (SEQ ID NO: 17; referred to as "PEP17" herein, e.g., JP 2003-000270 A); and
RYLTQETNKV (SEQ ID NO: 18; referred to as "PEP18" herein, e.g., WO 2005/116056).

Table 1 shows information concerning proteins from which the CTL epitope peptides PEP1 to PEP18 are derived. These proteins have been reported to be expressed at high levels in tumor tissue.

**[Table 1]**

| Peptide | Origin | Amino acid sequence | SEQ ID NO: |
|---|---|---|---|
| PEP1 | Lck-246 | KLVERLGAA | SEQ ID NO: 1 |
| PEP2 | WHSC2-103 | ASLDSDPWV | SEQ ID NO: 2 |
| PEP3 | HNRPL-140 | ALVEFEDVL | SEQ ID NO: 3 |
| PEP4 | SART3-302 | LLQAEAPRL | SEQ ID NO: 4 |
| PEP5 | SART2-93 | DYSARWNEI | SEQ ID NO: 5 |
| PEP6 | SART3-109 | VYDYNCHVDL | SEQ ID NO: 6 |
| PEP7 | MRP3-503 | LYAWEPSFL | SEQ ID NO: 7 |
| PEP8 | Lck-488 | DYLRSVLEDF | SEQ ID NO: 8 |
| PEP9 | SART3-734 | QIRPIFSNR | SEQ ID NO: 9 |
| PEP10 | Lck-90 | ILEQSGEWWK | SEQ ID NO: 10 |
| PEP11 | Lck-449 | VIQNLERGYR | SEQ ID NO: 11 |
| PEP12 | CypB-129 | KLKHYGPGWV | SEQ ID NO: 12 |
| PEP13 | UBE2V-43 | RLQEWCSVI | SEQ ID NO: 13 |
| PEP14 | WHSC2-141 | ILGELREKV | SEQ ID NO: 14 |
| PEP15 | EGFR-800 | DYVREHKDNI | SEQ ID NO: 15 |
| PEP16 | Lck-208 | HYTNASDGL | SEQ ID NO: 16 |
| PEP17 | MRP3-1293 | NYSVRYRPGL | SEQ ID NO: 17 |
| PEP18 | PTHr-102 | RYLTQETNKV | SEQ ID NO: 18 |

The peptide having 4 linked CTL epitopes preferably used in the present invention is a peptide comprising 4 types of CTL epitope peptides selected from among particular 13 types of the CTL epitope peptides: the peptide "PEP1" represented by SEQ ID NO: 1, the peptide "PEP2" represented by SEQ ID NO: 2, the peptide "PEP4" represented by SEQ ID NO: 4, the peptide "PEP5" represented by SEQ ID NO: 5, the peptide "PEP6" represented by SEQ ID NO: 6, the peptide "PEP7" represented by SEQ ID NO: 7, the peptide "PEP8" represented by SEQ ID NO: 8, the peptide "PEP9" represented by SEQ ID NO: 9, the peptide "PEP10" represented by SEQ ID NO: 10, the peptide "PEP13" represented by SEQ ID NO: 13, the peptide "PEP15" represented by SEQ ID NO: 15, the peptide "PEP17" represented by SEQ ID NO: 17, and the peptide "PEP18" represented by SEQ ID NO: 18, linearly linked via linkers. The peptide having 4 linked CTL epitopes can induce and/or activate three or more CTLs specific for relevant CTL epitope peptides. Peptides may not be directly evaluated concerning CTL epitope-peptide-specific induction. The occurrence of epitope-peptide-specific CTL induction can be determined by cleavage experiment with immunoproteasomes (e.g., WO 2015/060235).

In the present invention, a peptide having an amino acid sequence having substitution, insertion, deletion, and/or addition of one or a plurality of amino acids in the amino acid sequence of PEP1, PEP2, PEP4, PEP5, PEP6, PEP7, PEP8, PEP9, PEP10, PEP13, PEP15, PEP17, or PEP18 and having the capacity for inducing CTL and/or the capacity for inducting immunoglobulin production equivalent to or higher than those of the original peptide can be used as a "CTL epitope peptide." The term "plurality" used herein refers to 2 or 3, and preferably 2. An example of such peptide is a peptide obtained by substitution with amino acids having properties similar to those of the original amino acid (i.e., a peptide obtained by conservative amino acid substitution).

Whether or not a peptide of interest is a "peptide having the capacity for inducing CTL and/or the capacity for inducting immunoglobulin production equivalent to or higher than those of the original peptide" can be determined in accordance with, for example, the method disclosed in WO 2015/060235. According to the method disclosed in WO 2015/060235, the capacity for inducing CTL is evaluated using, as the indicator, the number of IFN-γ-producing cells in wells supplemented with cells obtained from a mouse to which a test peptide having an amino acid sequence having substitution, insertion, deletion, and/or addition of one or a plurality of amino acids has been administered in advance, antigen-presenting cells derived from a mouse of the same lineage, and the test peptide. When the result of evaluation is equivalent to or higher than the Δ value of the original peptide (positive: 10 ≤ Δ < 100; moderately positive: 100 ≤ Δ < 200; strongly positive: 200 ≤ Δ), the peptide of interest can be determined to have the capacity for inducing CTL equivalent to or higher than that of the original peptide. When the original peptide is evaluated "positive" and a peptide having an amino acid sequence having substitution, insertion, deletion, and/or addition of one or a plurality of amino acids is also evaluated "positive," the capacity for inducing CTL is considered equivalent. The capacity for inducting immunoglobulin production is evaluated using, as the indicator, the CTL-epitope-specific IgG antibody titer in the serum of the mouse to which the test peptide has been administered. When an increase in the obtained IgG antibody titer (fold) is equivalent to or higher than that of the original peptide (2 < fold < 10, 10 ≤ fold < 100, 100 ≤ fold), the peptide of interest can be determined to have the capacity for inducting immunoglobulin production equivalent to or higher than that of the original peptide. When the result of the measurement on the original peptide is within the range of "2 < fold < 10" and the result of the measurement on the peptide having an amino acid sequence having substitution, insertion, deletion, and/or addition of one or a plurality of amino acids is also within the range of "2 < fold < 10," the capacity for inducting immunoglobulin production is considered equivalent.

In the present invention, any linker can be used, provided that it is cleaved upon administration of a peptide having 4 linked CTL epitopes to an organism, and that the linked CTL epitope peptides can be separated from each other. Examples thereof include an ester bond, an ether bond, an amide bond, a sugar chain linker, a polyethylene glycol linker, and an amino acid linker. Examples of amino acid sequences used as amino acid linkers include an arginine dimer (RR), an arginine trimer (RRR), an arginine tetramer (RRRR), a lysine dimer (KK), a lysine trimer (KKK), a lysine tetramer (KKKK), a glycine dimer (GG), a glycine trimer (GGG), a glycine tetramer (GGGG), a glycine pentamer (GGGGG), a glycine hexamer (GGGGGG), alanine-alanine-tyrosine (AAY), isoleucine-leucine-alanine (ILA), and arginine-valine-lysine-arginine (RVKR), with an arginine dimer (RR) or trimer (RRR) being preferable and an arginine dimer (RR) being more preferable. Linkers used for an epitope-linked peptide are known in the art, and a person skilled in the art can use an adequately selected linker.

The peptide having 4 linked CTL epitopes that is preferably used in the present invention may further have a peptide sequence consisting of hydrophilic amino acids. A peptide sequence consisting of hydrophilic amino acids can be added to the N terminus and/or the C terminus of the peptide having 4 linked CTL epitopes, and it is preferably added to the N terminus. Such peptide sequence can comprise 1 to 15, preferably 2 to 10, and more preferably 3 to 5 hydrophilic amino acids selected from the group consisting of arginine, histidine, lysine, threonine, tyrosine, serine, asparagine, glutamine, aspartic acid, and glutamic acid. Examples of such peptide sequences consisting of hydrophilic amino acids include an arginine trimer (RRR), an arginine tetramer (RRRR), a lysine trimer (KKK), a lysine tetramer (KKKK), a histidine trimer (HHH), and a histidine tetramer (HHHH), with an arginine trimer (RRR) or tetramer (RRRR) being preferable and an arginine tetramer (RRRR) being more preferable.

The peptide having 4 linked CTL epitopes preferably used in the present invention is a peptide comprising 4 peptides each selected from the group consisting of: the peptide "PEP1" represented by SEQ ID NO: 1, the peptide "PEP2" represented by SEQ ID NO: 2, the peptide "PEP4" represented by SEQ ID NO: 4, the peptide "PEP5" represented by SEQ ID NO: 5, the peptide "PEP6" represented by SEQ ID NO: 6, the peptide "PEP7" represented by SEQ ID NO: 7, the peptide "PEP8" represented by SEQ ID NO: 8, the peptide "PEP9" represented by SEQ ID NO: 9, the peptide "PEP10" represented by SEQ ID NO: 10, the peptide "PEP13" represented by SEQ ID NO: 13, the peptide "PEP15" represented by SEQ ID NO: 15, the peptide "PEP17" represented by SEQ ID NO: 17, and the peptide "PEP18" represented by SEQ ID NO: 18, linked via linkers, which may have a peptide sequence consisting of hydrophilic amino acids, and which has a feature selected from among (1) to (3) below:
(1) the peptide comprises PEP2 at the C terminus, except for a peptide comprising PEP7 and PEP8 at the N terminus successively disposed via a linker in such order from the N terminus;
(2) the peptide comprises PEP4 at the C terminus; and
(3) the peptide comprises PEP10 at the C terminus.

The peptide having 4 linked CTL epitopes that is preferably used in the present invention is a peptide comprising 4 non-redundant peptides selected from the group consisting of the peptide "PEP1" represented by SEQ ID NO: 1, the peptide "PEP2" represented by SEQ ID NO: 2, the peptide "PEP4" represented by SEQ ID NO: 4, the peptide "PEP5" represented by SEQ ID NO: 5, the peptide "PEP6" represented by SEQ ID NO: 6, the peptide "PEP7" represented by SEQ ID NO: 7, the peptide "PEP8" represented by SEQ ID NO: 8, the peptide "PEP9" represented by SEQ ID NO: 9, the peptide "PEP10" represented by SEQ ID NO: 10, the peptide "PEP13" represented by SEQ ID NO: 13, the peptide "PEP15" represented by SEQ ID NO: 15, the peptide "PEP17" represented by SEQ ID NO: 17, and the peptide "PEP18" represented by SEQ ID NO: 18, linked via linkers, which may have a peptide sequence consisting of hydrophilic amino acids, and which has a feature selected from among (1) to (3) below:
(1) the peptide comprises 3 CTL epitope peptides selected from among PEP1, PEP7, PEP8, and PEP13, and comprises PEP2 at the C terminus, except for a peptide comprising PEP7 and PEP8 at the N terminus successively disposed via a linker in such order from the N terminus;
(2) the peptide comprises 3 CTL epitope peptides: PEP5, PEP6, and PEP9, and comprises PEP4 at the C terminus; and
(3) the peptide comprises 3 CTL epitope peptides selected from among PEP1, PEP13, PEP15, PEP17, and PEP18, and comprises PEP10 at the C terminus.

More preferably, the peptide having 4 linked CTL epitopes preferably used in the present invention comprises a sequence selected from among the following sequences, which may have a peptide sequence consisting of hydrophilic amino acids, wherein "-(L)-" represents a linker:
▪ PEP1-(L)-PEP7-(L)-PEP8-(L)-PEP2;
▪ PEP1-(L)-PEP8-(L)-PEP7-(L)-PEP2;
▪ PEP7-(L)-PEP1-(L)-PEP8-(L)-PEP2;
▪ PEP8-(L)-PEP1-(L)-PEP7-(L)-PEP2;
▪ PEP8-(L)-PEP7-(L)-PEP1-(L)-PEP2;
▪ PEP7-(L)-PEP13-(L)-PEP8-(L)-PEP2;
▪ PEP8-(L)-PEP7-(L)-PEP13-(L)-PEP2;
▪ PEP8-(L)-PEP13-(L)-PEP7-(L)-PEP2;
▪ PEP13-(L)-PEP7-(L)-PEP8-(L)-PEP2;
▪ PEP13-(L)-PEP8-(L)-PEP7-(L)-PEP2;
▪ PEP5-(L)-PEP6-(L)-PEP9-(L)-PEP4;
▪ PEP5-(L)-PEP9-(L)-PEP6-(L)-PEP4;
▪ PEP6-(L)-PEP5-(L)-PEP9-(L)-PEP4;
▪ PEP6-(L)-PEP9-(L)-PEP5-(L)-PEP4;
▪ PEP9-(L)-PEP5-(L)-PEP6-(L)-PEP4;
▪ PEP9-(L)-PEP6-(L)-PEP5-(L)-PEP4;
▪ PEP13-(L)-PEP15-(L)-PEP18-(L)-PEP10;
▪ PEP13-(L)-PEP18-(L)-PEP15-(L)-PEP10;
▪ PEP15-(L)-PEP13-(L)-PEP18-(L)-PEP10;
▪ PEP15-(L)-PEP18-(L)-PEP13-(L)-PEP10;
▪ PEP18-(L)-PEP13-(L)-PEP15-(L)-PEP10;
▪ PEP18-(L)-PEP15-(L)-PEP13-(L)-PEP10;
▪ PEP1-(L)-PEP15-(L)-PEP18-(L)-PEP10;
▪ PEP1-(L)-PEP18-(L)-PEP15-(L)-PEP10;
▪ PEP15-(L)-PEP1-(L)-PEP18-(L)-PEP10;
▪ PEP15-(L)-PEP18-(L)-PEP1-(L)-PEP10;
▪ PEP18-(L)-PEP1-(L)-PEP15-(L)-PEP10;
▪ PEP18-(L)-PEP15-(L)-PEP1-(L)-PEP10;
▪ PEP13-(L)-PEP15-(L)-PEP17-(L)-PEP10;
▪ PEP13-(L)-PEP17-(L)-PEP15-(L)-PEP10;
▪ PEP15-(L)-PEP13-(L)-PEP17-(L)-PEP10;
▪ PEP15-(L)-PEP17-(L)-PEP13-(L)-PEP10;
▪ PEP17-(L)-PEP13-(L)-PEP15-(L)-PEP10; and
▪ PEP17-(L)-PEP15-(L)-PEP13-(L)-PEP10.

In the peptide having 4 linked CTL epitopes preferably used in the present invention, more preferably, the linker represented by (L) in the above sequence is an amino acid linker, and the peptide may have a peptide sequence consisting of hydrophilic amino acids.

In the peptide having 4 linked CTL epitopes that is preferably used in the present invention, more preferably, the linker represented by (L) in the above sequence is an arginine dimer or trimer comprising 2 or 3 arginine residues linked to each other, and the peptide may have a peptide sequence consisting of hydrophilic amino acids.

In the peptide having 4 linked CTL epitopes preferably used in the present invention, more preferably, the linker represented by (L) in the above sequence is an arginine dimer or trimer comprising 2 or 3 arginine residues linked to each other, and the peptide may have a peptide sequence consisting of an arginine trimer or tetramer comprising 3 or 4 linked arginine residues at the N terminus as hydrophilic amino acids.

The peptide having 4 linked CTL epitopes preferably used in the present invention can be synthesized in accordance with, for example, the method disclosed in WO 2015/060235.

In the present invention, a solution of peptides having 4 linked CTL epitopes used to prepare an emulsion formulation contains 3 types of peptides having 4 linked CTL epitopes.

When an emulsion formulation contains 3 types of peptides having 4 linked CTL epitopes in the present invention, preferably, 3 types of peptides having 4 linked CTL epitopes are composed of a peptide represented by Formula (I), a peptide represented by Formula (II), and a peptide represented by Formula (III):
A-(L)-B-(L)-C-(L)-PEP2 (I);
D-(L)-E-(L)-F-(L)-PEP4 (II); and
G-(L)-H-(L)-I-(L)-PEP10 (III),
wherein (L) represents a linker; and A, B, C, D, E, F, G, H, and I are each independently selected from the group consisting of PEP1, PEP5, PEP6, PEP7, PEP8, PEP9, PEP13, PEP15, and PEP18 (except for a combination of A representing PEP7 and B representing PEP8); and
the peptides having 4 linked CTL epitopes may each have a peptide sequence consisting of hydrophilic amino acids.

The 3 types of peptides having 4 linked CTL epitopes that are more preferably used in the present invention are composed of a peptide represented by Formula (I), a peptide represented by Formula (II), and a peptide represented by Formula (III), wherein (L) represents a linker,
the peptide represented by Formula (I) is selected from the group consisting of the following:
   ▪ PEP7-(L)-PEP13-(L)-PEP8-(L)-PEP2;
   ▪ PEP8-(L)-PEP7-(L)-PEP13-(L)-PEP2;
   ▪ PEP8-(L)-PEP13-(L)-PEP7-(L)-PEP2;
   ▪ PEP13-(L)-PEP7-(L)-PEP8-(L)-PEP2; and
   ▪ PEP13-(L)-PEP8-(L)-PEP7-(L)-PEP2,
the peptide represented by Formula (II) is selected from the group consisting of the following:
   ▪ PEP5-(L)-PEP6-(L)-PEP9-(L)-PEP4;
   ▪ PEP5-(L)-PEP9-(L)-PEP6-(L)-PEP4;
   ▪ PEP6-(L)-PEP5-(L)-PEP9-(L)-PEP4;
   ▪ PEP6-(L)-PEP9-(L)-PEP5-(L)-PEP4;
   ▪ PEP9-(L)-PEP5-(L)-PEP6-(L)-PEP4; and
   ▪ PEP9-(L)-PEP6-(L)-PEP5-(L)-PEP4, and
the peptide represented by Formula (III) is selected from the group consisting of the following:
   ▪ PEP1-(L)-PEP15-(L)-PEP18-(L)-PEP10;
   ▪ PEP1-(L)-PEP18-(L)-PEP15-(L)-PEP10;
   ▪ PEP15-(L)-PEP1-(L)-PEP18-(L)-PEP10;
   ▪ PEP15-(L)-PEP18-(L)-PEP1-(L)-PEP10;
   ▪ PEP18-(L)-PEP1-(L)-PEP15-(L)-PEP10; and
   ▪ PEP18-(L)-PEP15-(L)-PEP1-(L)-PEP10.
The peptide represented by Formula (I) is a peptide having 4 linked CTL epitopes, which may have a peptide sequence consisting of hydrophilic amino acids at the N terminus and/or the C terminus.

The 3 types of peptides having 4 linked CTL epitopes that are more preferably used in the present invention are composed of a peptide represented by Formula (I), a peptide represented by Formula (II), and a peptide represented by Formula (III), wherein (L) represents a linker,
the peptide represented by Formula (I) is selected from the group consisting of the following:
   ▪ PEP7-(L)-PEP13-(L)-PEP8-(L)-PEP2;
   ▪ PEP8-(L)-PEP7-(L)-PEP13-(L)-PEP2;
   ▪ PEP8-(L)-PEP13-(L)-PEP7-(L)-PEP2;
   ▪ PEP13-(L)-PEP7-(L)-PEP8-(L)-PEP2; and
   ▪ PEP13-(L)-PEP8-(L)-PEP7-(L)-PEP2,
the peptide represented by Formula (II) is selected from the group consisting of the following:
   ▪ PEP5-(L)-PEP6-(L)-PEP9-(L)-PEP4;
   ▪ PEP5-(L)-PEP9-(L)-PEP6-(L)-PEP4;
   ▪ PEP6-(L)-PEP5-(L)-PEP9-(L)-PEP4;
   ▪ PEP6-(L)-PEP9-(L)-PEP5-(L)-PEP4;
   ▪ PEP9-(L)-PEP5-(L)-PEP6-(L)-PEP4; and
   ▪ PEP9-(L)-PEP6-(L)-PEP5-(L)-PEP4, and
the peptide represented by Formula (III) is selected from the group consisting of the following:
   ▪ PEP1-(L)-PEP15-(L)-PEP18-(L)-PEP10;
   ▪ PEP1-(L)-PEP18-(L)-PEP15-(L)-PEP10;
   ▪ PEP15-(L)-PEP1-(L)-PEP18-(L)-PEP10;
   ▪ PEP15-(L)-PEP18-(L)-PEP1-(L)-PEP10;
   ▪ PEP18-(L)-PEP1-(L)-PEP15-(L)-PEP10; and
   ▪ PEP18-(L)-PEP15-(L)-PEP1-(L)-PEP10, and
wherein the peptide represented by Formula (I) has, as a peptide sequence consisting of hydrophilic amino acids, an arginine trimer or tetramer at the N terminus.

A more preferable combination of the 3 types of peptides having 4 linked CTL epitopes used in the present invention is composed of RRRR-PEP7-(L)-PEP13-(L)-PEP8-(L)-PEP2, PEP5-(L)-PEP9-(L)-PEP6-(L)-PEP4, and PEP15-(L)-PEP18-(L)-PEP1-(L)-PEP10, wherein (L) represents a linker.

The most preferable combination of the 3 types of peptides having 4 linked CTL epitopes used in the present invention is composed of RRRR-PEP7-RR-PEP13-RR-PEP8-RR-PEP2 (SEQ ID NO: 19), PEP5-RR-PEP9-RR-PEP6-RR-PEP4 (SEQ ID NO: 25), and PEP15-RR-PEP18-RR-PEP1-RR-PEP10 (SEQ ID NO: 33).

According to an embodiment of the present invention, the 3 types of peptides having 4 linked CTL epitopes are composed of:
a peptide represented by Formula (I'): A-RR-B-RR-C-RR-PEP2;
a peptide represented by Formula (II'): D-RR-E-RR-F-RR-PEP4; and
a peptide represented by Formula (III'): G-RR-H-RR-I-RR-PEP10,
wherein RR represents an arginine dimer; and A, B, C, D, E, F, G, H, and I are each independently selected from the group consisting of PEP1, PEP5, PEP6, PEP7, PEP8, PEP9, PEP13, PEP15, and PEP18 (except for a combination of A representing PEP7 and B representing PEP8), and
one of the peptides having 4 linked CTL epitopes represented by Formulae (I'), (II'), or (III') has an arginine tetramer at the N terminus.

The 3 types of peptides having 4 linked CTL epitopes that are more preferably used in the present invention are composed of a peptide represented by Formula (I') above, a peptide represented by Formula (II') above, and a peptide represented by Formula (III') above,
the peptide represented by Formula (I') is selected from the group consisting of the following:
   ▪ RRRR-PEP7-RR-PEP13-RR-PEP8-RR-PEP2 (SEQ ID NO: 19);
   ▪ RRRR-PEP8-RR-PEP7-RR-PEP13-RR-PEP2 (SEQ ID NO: 20);
   ▪ RRRR-PEP8-RR-PEP13-RR-PEP7-RR-PEP2 (SEQ ID NO: 21);
   ▪ RRRR-PEP13-RR-PEP7-RR-PEP8-RR-PEP2 (SEQ ID NO: 22); and
   ▪ RRRR-PEP13-RR-PEP8-RR-PEP7-RR-PEP2 (SEQ ID NO: 23),
the peptide represented by Formula (II') is selected from the group consisting of the following:
   ▪ PEP5-RR-PEP6-RR-PEP9-RR-PEP4 (SEQ ID NO: 24);
   ▪ PEP5-RR-PEP9-RR-PEP6-RR-PEP4 (SEQ ID NO: 25);
   ▪ PEP6-RR-PEP5-RR-PEP9-RR-PEP4 (SEQ ID NO: 26);
   ▪ PEP6-RR-PEP9-RR-PEP5-RR-PEP4 (SEQ ID NO: 27);
   ▪ PEP9-RR-PEP5-RR-PEP6-RR-PEP4 (SEQ ID NO: 28); and
   ▪ PEP9-RR-PEP6-RR-PEP5-RR-PEP4 (SEQ ID NO: 29), and
the peptide represented by Formula (III') is selected from the group consisting of the following:
   ▪ PEP1-RR-PEP15-RR-PEP18-RR-PEP10 (SEQ ID NO: 30);
   ▪ PEP1-RR-PEP18-RR-PEP15-RR-PEP10 (SEQ ID NO: 31);
   ▪ PEP15-RR-PEP1-RR-PEP18-RR-PEP10 (SEQ ID NO: 32);
   ▪ PEP15-RR-PEP18-RR-PEP1-RR-PEP10 (SEQ ID NO: 33);
   ▪ PEP18-RR-PEP1-RR-PEP15-RR-PEP10 (SEQ ID NO: 34); and
   ▪ PEP18-RR-PEP15-RR-PEP1-RR-PEP10 (SEQ ID NO: 35),
wherein RR represents an arginine dimer; and RRRR represents an arginine tetramer.

In the present invention, an aqueous phase comprising peptides having 4 linked CTL epitopes can be a solution comprising 3 types of lyophilized peptides having 4 linked CTL epitopes dissolved with solubilizer. When lyophilizing peptides having 4 linked CTL epitopes, trehalose or other excipients can be added. Thus, the aqueous phase comprising peptides having 4 linked CTL epitopes may contain trehalose or other excipients. A solution for injection can be used as the solution. Examples include an injection solvent and physiological saline, with an injection solvent being preferable. The concentration of 3 types of peptides having 4 linked CTL epitopes in the aqueous phase in total is preferably 3 to 27 mg/ml, more preferably 6 to 18 mg/ml, further preferably 7 to 12 mg/ml, and still further preferably 9 mg/ml.

The water-in-oil (w/o) emulsion formulation comprising the peptides having 4 linked epitopes of the present invention in the aqueous phase comprises, in terms of 3 types of peptides having 4 linked CTL epitopes in total, preferably 3 to 27 mg, more preferably 6 to 18 mg, further preferably 7 to 12 mg, and still further preferably 9 mg, per dose.

An average particle size of particles of the water-in-oil (w/o) emulsion formulation comprising the peptides having 4 linked epitopes of the present invention in the aqueous phase is preferably 1 to 3 µm. More preferably, an average particle size is 1 to 3 µm, and preferably 90% or more, more preferably 95% or more, and further preferably 99% or more of the particles in the emulsion formulation are within the range of particle size of 1 to 3 µm.

The viscosity of the water-in-oil (w/o) emulsion formulation comprising the peptides having 4 linked epitopes of the present invention in the aqueous phase is not particularly limited, and preferably 200 to 400 mPa·S.

In the present invention, an oil adjuvant is not particularly limited, provided that it forms a w/o emulsion when mixed with an aqueous phase and emulsified and that it is an oil immunoadjuvant that can be administered in the form of an emulsion and accelerate immune responses. Examples of oil adjuvants include an adjuvant consisting of an oil component and an adjuvant comprising an oil component and a surfactant. Examples of oil adjuvants preferably used in the present invention include liquid paraffin, lanolin, squalene, Freund's incomplete adjuvant, Freund's complete adjuvant, NH₂ (Cancer Sci., 101 (10): 2110-2114, 2010), Montanide ISA 720 VG, and Montanide ISA 51 VG. In the present invention, a particularly preferable oil adjuvant is Montanide ISA 51 VG, which is available from, for example, SEPPIC.

An oil adjuvant can be used alone as an oil phase to prepare an emulsion, or can be used in the form of a mixture with other oil components and/or surfactant to serve as an oil phase. For example, Montanide ISA 51 VG mentioned above can be used alone as an oil phase to prepare a w/o emulsion formulation.

By preparing a w/o emulsion, an antigen dissolved in an aqueous phase can remain at the administration site for a long period of time. In addition, the antigen is protected by an oil phase, so that it can be protected from enzymatic degradation. Further, the antigen enclosed in a water droplet of a given size or larger is more likely to be phagocytized by an immunocyte, compared with an antigen by itself, and its immunogenicity can be enhanced. Such properties cannot be achieved with an o/w emulsion, and it is thus very important to prepare the cancer vaccine of the present invention in the form of a w/o emulsion.

When preparing the emulsion formulation of the present invention, the amount of an oil phase comprising an oil adjuvant is preferably 0.5 to 2 fold (v/v), and more preferably equivalent to that of an aqueous phase comprising peptides having 4 linked CTL epitopes.

### <A method for preparing a w/o emulsion>

The present invention also provides a method for preparing the emulsion formulation according to the present invention comprising the steps (i) and (ii):
(i) a step of injecting a part of an aqueous phase comprising peptides having 4 linked CTL epitopes into an oil phase comprising an oil adjuvant to prepare a mixture; and
(ii) a step of allowing the mixture to reciprocate between syringes connected via a syringe connector,
wherein the step (i) and the step (ii) are repeated to obtain an emulsion formulation comprising the total amount of the oil phase and the aqueous phase.

In the present invention, the amount "a part" is preferably 1/10 to 1/2 fold (v/v), more preferably 1/10 to 2/5 fold (v/v), further preferably 1/10 to 1/5 fold (v/v), and the most preferably 1/5 fold (v/v), relative to the amount of an oil phase comprising an oil adjuvant. When the amount of an oil adjuvant is 1 ml, for example, "a part" of "an aqueous phase comprising peptides having 4 linked CTL epitopes" is preferably 0.1 to 0.4 ml, more preferably 0.1 to 0.2 ml, and the most preferably 0.2 ml.

The term "main emulsification" used herein refers to an operation of emulsification that is carried out after the total amount of an oil phase and the total amount of an aqueous phase are mixed. The term "pre-emulsification" refers to an operation of emulsification that is carried out at every instance of mixing of a part of the aqueous phase with the total amount of the oil phase. The operation of emulsification that is carried out after the final operation of such mixing of the aqueous phase to the total amount of the oil phase is regarded as "main emulsification", since the total amount of the oil phase has been mixed with the total amount of the aqueous phase.

The emulsion formulation according to the present invention can be produced by the method disclosed in, for example, WO 2007/083763. According to a conventional standard technique of emulsion preparation, however, pre-emulsification can be carried out only by shaking a syringe. Accordingly, it is the most preferable that the emulsion formulation according to the present invention be prepared with the use of the apparatus of the present invention in accordance with the method of the present invention described below.

Specifically, the emulsion formulation according to the present invention can be prepared by allowing a mixture of an oil phase comprising an oil adjuvant and an aqueous phase comprising peptides having 4 linked CTL epitopes to reciprocate between 2 syringes connected via a syringe connector with a narrow fluid channel (e.g., inner diameter: 0.8 to 1.7 mm) to emulsify the mixture (including pre-emulsification and main emulsification). An example of such syringe connector is a GP syringe connector (Nipro Corporation).

When performing pre-emulsification and main emulsification in the present invention, a mixture of an oil phase comprising an oil adjuvant and an aqueous phase comprising peptides having 4 linked CTL epitopes is allowed to reciprocate between syringes connected via a syringe connector, for example, 10 times or more, 20 times or more, 30 times or more, 40 times or more, 50 times or more, or 60 times. From the viewpoint of operation efficiency, the number of reciprocation at the time of pre-emulsification is preferably 10 to 20, and more preferably 10. The number of reciprocation at the time of main emulsification is preferably 10 to 20.

In the present invention, the method for preparing a w/o emulsion formulation comprising peptides having 4 linked CTL epitopes comprises the following steps:
(i) a step of injecting a part of an aqueous phase comprising peptides having 4 linked CTL epitopes into an oil phase comprising an oil adjuvant to prepare a mixture; and
(ii) a step of allowing the mixture to reciprocate between syringes connected via a syringe connector.

For example, the above method comprises the following steps (i) and (ii):
(i) a step of injecting a part of an aqueous phase comprising peptides having 4 linked CTL epitopes into an oil phase comprising an oil adjuvant to prepare a mixture; and
(ii) a step of allowing the mixture to reciprocate between syringes connected via a syringe connector,
wherein the step (i) and the step (ii) are repeated to obtain an emulsion formulation comprising the total amount of the oil phase and the aqueous phase.

In such a case, the amount of a part of the aqueous phase can be preferably 1/10 to 1/2 fold (v/v), more preferably 1/10 to 2/5 fold (v/v), further preferably 1/10 to 1/5 fold (v/v), and the most preferably 1/5 fold (v/v), relative to the amount of an oil phase comprising an oil adjuvant.

The step (i) and the step (ii) can be repeated 2 to 10 times, preferably 3 to 7 times, and more preferably 5 times.

The method for preparing a w/o emulsion formulation comprising peptides having 4 linked CTL epitopes preferably comprises the following step (i) and step (ii):
(i) a step of injecting a part of an aqueous phase comprising peptides having 4 linked CTL epitopes into an oil phase comprising an oil adjuvant in a step-wise manner in an amount of 1/5 fold (v/v) that of the oil phase and repeating such injection 5 times in total to prepare a mixture; and
(ii) a step of allowing the mixture to reciprocate between syringes connected via a syringe connector at every instance of the injection.

The method for preparing a w/o emulsion formulation comprising peptides having 4 linked CTL epitopes more preferably comprises the following steps (i) to (iv):
(i) a step of injecting an aqueous phase comprising peptides having 4 linked CTL epitopes into an oil phase comprising an oil adjuvant in an amount of 1/5 (v/v) that of the oil phase to prepare a mixture;
(ii) a step of allowing the mixture to reciprocate between syringes connected via a syringe connector 10 times or more after the injection;
(iii) a step of repeating the step (i) and the step (ii) 4 more times; and
(iv) a step of allowing the mixture to reciprocate between syringes connected via a syringe connector 10 times or more.

The method for preparing a w/o emulsion formulation comprising peptides having 4 linked CTL epitopes more preferably comprises the following steps (i) to (iv):
(i) a step of injecting a solution of peptides having 4 linked CTL epitopes into Montanide ISA 51 VG in an amount of 1/5 fold (v/v) that of Montanide ISA 51 VG to prepare a mixture;
(ii) a step of allowing the mixture to reciprocate between syringes connected via a syringe connector 10 times or more after the injection;
(iii) a step of repeating the step (i) and the step (ii) 4 more times; and
(iv) a step of allowing the mixture to reciprocate between syringes connected via a syringe connector 10 times or more.

The method for preparing the emulsion formulation of peptides having 4 linked CTL epitopes preferably comprises an additional step of confirming a type of the resulting emulsion by a drop test. In the present invention, a drop test comprises to drop a droplet of the resulting emulsion on a water surface to confirm if the dropped emulsion is dispersed or not. When the emulsion is not immediately dispersed in water by the drop test, the prepared emulsion can be determined as a "water-in-oil (w/o) emulsion". When the emulsion is immediately dispersed in water, the prepared emulsion can be determined as an "oil-in-water (o/w) emulsion." In the present invention, it is necessary to obtain a w/o emulsion.

### <Apparatus>

In the present invention, an apparatus that can be preferably used to prepare an emulsion formulation of peptides having 4 linked CTL epitopes comprises, for example, a three-way stopcock, and 2 syringes and a syringe connector connected to the three-way stopcock, and the syringes are connected to the syringe connector that is connected to the three-way stopcock.

As described above, the emulsion formulation is to be prepared at the time of use, and mass-production is not suitable because of a small amount thereof to be administered. Thus, an emulsion formulation is preferably prepared with the use of a syringe with a volume of several ml, such as an injection syringe. In the past, a method for preparing such emulsion formulation involving the use of an apparatus comprising 2 syringes connected to each other via a connector had been known (WO 2007/083763; JP Patent No. 5,629,882).

The emulsion formulation according to the present invention can be prepared using the conventional apparatus as described above. With the use of the conventional apparatus, however, pre-emulsification can be carried out only by shaking a syringe, and the results of pre-emulsification are easily affected by a method or force of shaking. Accordingly, it is difficult to determine as to whether or not sufficient shaking for pre-emulsification was performed on the basis of the time. In addition, whether or not sufficient pre-emulsification had been performed is determined by a person who had prepared an emulsion based on his/her subjective view. Accordingly, there would be variations between/among persons who had prepared emulsions and that there would be variations at every instance of preparation by the same person.

When a conventional apparatus is used, in addition, the procedure for preparation is time-consuming, preparation of a stable w/o emulsion may result in a failure, and workers are seriously burdened in clinical situations such as hospitals where emulsion formulations are to be prepared at the time use. Such drawbacks are desired to be dissolved.

To this end, the present inventors discovered that the method of the present invention could be readily realized by connecting, via a three-way stopcock, a third syringe to the 2 syringes connected to each other to prepare an emulsion formulation at the time of use.

An apparatus preferably used in the method of the present invention comprises, as shown in Fig. 1, a first syringe 1, a second syringe 2 connected to the first syringe 1 via a syringe connector 5, and a third syringe 3 further connected to the first syringe 1 and the second syringe 2 via a three-way stopcock 4.

Specifically, the present invention provides an apparatus used for preparing a w/o emulsion formulation containing peptides, which is composed of:
a second syringe 2;
a syringe connector 5 connected to the second syringe 2;
a three-way stopcock 4 connected to the syringe connector 5; and
a first syringe 1 and a third syringe 3 further connected to the three-way stopcock 4,
wherein the apparatus enables production of a w/o emulsion formulation by adding an aqueous phase filled in the third syringe 3 to the oil phase filled in the first syringe 1 or the second syringe 2 and allowing the resulting mixture to reciprocate between the first syringe 1 and the second syringe 2 via the syringe connector 5.

While the volumes of the first, the second, and the third syringes are not particularly limited, for example, each volume is preferably adjusted to 3 ml or less from the viewpoint of ease of operation at the time of preparation. As described above, the w/o emulsion formulation of the present invention is generally prepared at the time of use. Thus, the amount thereof to be prepared before administration is small, and the necessity of mass-production is insignificant.

In the apparatus of the present invention, an inner diameter of the syringe connector 5 through which the mixture passes is preferably 0.8 to 1.7 mm.

The method for preparation using such apparatus can yield the effects as described below.
(1) According to a conventional method for preparing an emulsion, pre-emulsification was performed by shaking a syringe, and the conditions of an aqueous phase mixed with an oil phase were thus likely to vary between/among instances of preparation. According to the present invention, in contrast, the emulsion is allowed to pass through a syringe connector with a narrow inner diameter at the time of pre-emulsification in addition to the time of main emulsification, which provides a shearing force, and a variation in the conditions of an aqueous phase mixed with an oil phase becomes small between/among instances of preparation.
(2) Fluid channels can be switched via introduction of a three-way stopcock, an aqueous phase is divided into sections, each section is separately injected into the oil phase, the oil-rich conditions are realized, and pre-emulsification via reciprocal movement of a syringe plunger can be performed.

A cross sectional area (a broken line B in Figs. 1 and 2) inside the syringe cylinder which may be used for the apparatus of the present invention is preferably 2 to 150 times and more preferably 2 to 50 times larger than a cross sectional area (a broken line A in Figs. 1 and 2) of a fluid channel in a syringe connector. The cross sectional area inside the syringe cylinder should be larger by at least 2 times than the cross sectional area of a fluid channel in a syringe connector, otherwise the effects as described above may not be satisfactorily exerted. The cross sectional area inside the syringe cylinder should not be larger by over 150 times than the cross sectional area of a fluid channel in a syringe connector, otherwise excessive stress may be imposed on the syringe, the apparatus, and the like.

More specifically, the cross sectional area inside the syringe cylinder may be approximately 50 to 500 mm², and the cross sectional area of a fluid channel in a syringe connector may be approximately 0.5 to 30 mm². While the shape of the cross section of the syringe cylinder or the syringe connector is not particularly limited, a round, oval, or approximately round polygonal shape is preferable, so as to prevent topical stress, with a round shape being particularly preferable. The term "cross sectional area" used herein refers to a cross sectional area inside the syringe cylinder or a cross sectional area of the narrowest region of the fluid channel formed in the syringe connector.

The syringe connector used in the present invention needs to be in close contact with the syringe at its tip. As shown in Figs. 1 and 2, accordingly, the syringe connector tip 6 may be formed to have a cross section conically increasing from the fluid channel in the middle.

When a cross section of the fluid channel of the syringe connector is made round, the inner diameter of the fluid channel is preferably 0.8 to 1.7 mm and more preferably 0.9 to 1.5 mm. When the thickness of the fluid channel is not uniform, the inner diameter of the narrowest region is regarded as the inner diameter of the fluid channel. The length of the fluid channel in the syringe connector is preferably 10 to 15 mm. An example of such syringe connector is a GP syringe connector (Nipro Corporation).

The apparatus can be operated manually by healthcare professionals, for example, before administration of the emulsion formulation. Alternatively, the apparatus can be an automated apparatus that is constituted, in accordance with the technique in the art as disclosed in, for example, JP Patent No. 5,629,882, to comprise a fixing mechanism fixing the first, the second, and the third syringes, the first, the second, and the third push mechanisms pushing plungers of the first, the second, and the third syringes, respectively, and a control mechanism controlling such push mechanisms.

Another aspect of the present invention can provide a kit used for preparation of a w/o emulsion comprising peptides having 4 linked CTL epitopes, which comprises peptides having 4 linked CTL epitopes each comprising 4 CTL epitope peptides linked via linkers, an oil adjuvant, and the apparatus described above.

Target tumors to be treated in a patient to whom the emulsion formulation according to the present invention is administered are not particularly limited, provided that anti-tumor effects can be enhanced. Target tumors are preferably tumors on which the peptides having 4 linked CTL epitopes exert anti-tumor effects and more preferably Lck-, WHSC2-, SART2-, SART3-, MRP3-, UBE2V-, EGFR-, or PTHrP-positive malignant tumors.

Specific examples of target tumors to be treated with the use of the emulsion formulation according to the present invention include brain tumor, head and neck cancer, digestive system cancer (e.g., esophageal cancer, gastric cancer, duodenal cancer, liver cancer, bile duct cancer (e.g., gallbladder cancer and bile duct cancer), pancreatic cancer, small intestinal cancer, large bowel cancer (e.g., colorectal cancer, colon cancer, and rectal cancer), and gastrointestinal stromal tumor), lung cancer (e.g., non-small cell lung cancer and small cell lung cancer), breast cancer, ovarian cancer, uterine cancer (e.g., cervical cancer and uterine body cancer), renal cancer, urothelial cancer (e.g., bladder cancer, renal pelvic cancer, and ureteral cancer), prostate cancer, skin cancer, and cancer of unknown primary. Cancer include primary cancer and cancer metastasized to other organs (e.g., liver). From the viewpoint of anti-tumor effects, preferable targets are head and neck cancer, digestive system cancer, lung cancer, renal cancer, urothelial cancer, and skin cancer, more preferable targets are digestive system cancer, lung cancer, urothelial cancer, and skin cancer, and particularly preferable targets are lung cancer and urothelial cancer. The anti-tumor agent of the present invention may be used for post-operative adjuvant chemotherapy performed for recurrence prevention after surgical tumor resection or pre-operative adjuvant chemotherapy performed before surgical tumor resection.

In the present invention, the w/o emulsion formulation comprising peptides having 4 linked CTL epitopes may comprise pharmaceutical carriers, according to need, in addition to the peptides having 4 linked CTL epitopes as active ingredients, and can be administered in the form of an injection.

Examples of pharmaceutical carriers include various types of carriers that are commonly used for medicines, such as an excipient, a solvent, a solubilizer, a suspending agent, an isotonizing agent, a pH modifier, a buffer, a stabilizer, and a soothing agent. According to need, additives, such as a preservative, an anti-oxidant, a colorant, and a flavoring agent, can also be used.

Examples of excipients include lactose, sucrose, D-mannitol, and trehalose.

Examples of solvents include water, propylene glycol, and physiological saline.

Examples of solubilizers include polyethylene glycol, ethanol, α-cyclodextrin, macrogol 400, and polysorbate 80.

An example of a suspending agent is polyoxyethylene hydrogenated castor oil.

Examples of isotonizing agents include sodium chloride, glycerin, and potassium chloride.

Examples of pH modifiers include sodium citrate, sodium hydroxide, and hydrochloric acid.

Examples of buffers include disodium hydrogen phosphate and sodium dihydrogen phosphate.

Examples of antioxidants include sodium sulfite, ascorbic acid, natural vitamin E, and methionine.

The emulsion formulation according to the present invention is administered in the form of an injection, preferably intravenously. The amount of each type of the peptides having 4 linked CTL epitopes to be incorporated in a dosage unit form is preferably 1 to 9 mg, more preferably 2 to 4.5 mg, and the most preferably 3 mg.

A daily dose of each type of the peptides having 4 linked CTL epitopes in the dosage form described above varies depending on the symptoms, body weight, age, sexuality, and other conditions of a patient, and it cannot be generally determined. A daily dose is preferably 1 to 27 mg/body, more preferably 2 to 12 mg/body, and further preferably 3 to 9 mg/body. Administration is preferably carried out once or 2 or 3 separate instances a day.

The schedule for administration of the peptides having 4 linked CTL epitopes of the present invention can be adequately determined in accordance with a cancer type, the disease stage, and other conditions.

The administration is preferably scheduled to comprise a cycle of 21 days in total in which a step of single administration per week is repeated 3 times (once a day on Day 1, Day 8, and day 15). After the third cycle, administration is preferably scheduled to comprise a cycle of 21 days in total in which drug administration on Day 1 is followed by drug holidays for 20 days (single administration in 3 weeks).

### [Examples]

Hereafter, the present invention is described in greater detail with reference to the examples. It should be noted that the following examples involve the use of an apparatus of a particular constitution and that the present invention is not limited to these examples.

In the following examples, the solution of peptides having 4 linked CTL epitopes contains, as a peptide having 4 linked CTL epitopes, three types of peptides consisting of a peptide represented by SEQ ID NO: 19, a peptide represented by SEQ ID NO: 25, and a peptide represented by SEQ ID NO: 33, each at 3 mg/mL, unless otherwise specified.

### [Example 1: Examination of the number of divisional mixing of an aqueous phase with an oil phase at the time of pre-emulsification]

A w/o emulsion was prepared using a GP syringe connector (inner diameter: 1.0 mm; fluid channel length: 15 mm, Nipro Corporation) in accordance with a standard method of emulsion preparation described in the instructions of the GP syringe connector.

Specifically, syringes each filled with 1 ml of a solution of peptides having 4 linked CTL epitopes and Montanide ISA 51 VG (SEPPIC) were prepared (Injekt Syringes, 2 ml, Luer Lock, B. Braun), and the syringes were connected to each other via a GP syringe connector while preventing air inclusion. The total amount of the solution of peptides having 4 linked CTL epitopes (the aqueous phase) was injected into the syringe containing Montanide ISA 51 VG (the oil phase) (without division), and the syringes were vigorously shaken for approximately 5 to 10 minutes to perform pre-emulsification. Pre-emulsification was terminated when no clear water droplets or oil droplets were observed in the solution and a homogeneous white turbid liquid was obtained.

Thereafter, main emulsification was performed to prepare a w/o emulsion by alternately pushing the syringe plungers to allow the mixture to reciprocate between syringes 60 times. Emulsion preparation was performed 5 times, and the rate of the w/o emulsion prepared was determined by a drop test. When the prepared emulsion was not easily dispersed in water, the emulsion of interest was evaluated as a "w/o emulsion." When the prepared emulsion was easily dispersed in water, the emulsion of interest was evaluated as a "o/w emulsion."

As shown in Table 2, the rate of the w/o emulsion containing the solution of peptides having 4 linked CTL epitopes prepared by the standard method of emulsion preparation was found to be 0%, and it was found very difficult to prepare a w/o emulsion containing the solution of peptides having 4 linked CTL epitopes by the standard method of emulsion preparation.

In order to solve the problems described above, a method in which a solution of peptides having 4 linked CTL epitopes (the aqueous phase) was divided and mixed with Montanide ISA 51 VG (the oil phase) (a method of divisional preparation) was examined. In the standard method of emulsion preparation, specifically, a solution of peptides having 4 linked CTL epitopes (the aqueous phase) was divided into 3 or 5 sections, the sections were separately injected into the syringe containing Montanide ISA 51 VG (the oil phase), and the syringe was vigorously shaken to perform pre-emulsification. Pre-emulsification was terminated when no clear water droplets or oil droplets were observed in the liquid and a homogeneous white turbid liquid was obtained (the syringe was vigorously shaken for approximately 5 to 10 minutes per single pre-emulsification operation).

Thereafter, main emulsification was performed to prepare a w/o emulsion by alternately pushing the syringe plungers to allow the mixture to reciprocate between syringes 60 times. Emulsion preparation was performed 5 times separately, and the rate of the w/o emulsion obtained was determined by a drop test.

**[Table 2]**

| Division | Not divided | Divided into 3 | Divided into 5 |
|---|---|---|---|
| Amount of aqueous phase injected into oil phase per operation (mL) | 1 | 0.33 | 0.2 |
| Rate of w/o emulsion obtained (%) | 0 | 20 | 100 |

As shown in Table 2, the rate of the w/o emulsion obtained was increased by dividing the solution of peptides having 4 linked CTL epitopes (the aqueous phase) into 3 sections at the time of pre-emulsification, and the rate of the w/o emulsion obtained was significantly increased by dividing the solution of peptides having 4 linked CTL epitopes (the aqueous phase) into 5 sections, compared with the case in which the solution was not divided. It was found preferable that the aqueous phase be divided into higher numbers of sections and injected into the oil phase to prepare a w/o emulsion; i.e., a w/o emulsion be prepared under the oil-rich conditions.

### [Example 2: Preparation of an emulsion via divisional injection of a solution of peptides having 4 linked CTL epitopes]

In the method of dividing the solution of peptides having 4 linked CTL epitopes into 5 sections and divisionally injecting the same into the oil phase described in Example 1, the number of persons and the number of instances to prepare emulsions were increased, and the rate of the w/o emulsion obtained was examined. In order to reduce variations among instances of pre-emulsification, the syringes were vigorously shaken with the use of YS-8D (Yayoi Co., Ltd.) in pre-emulsification. When pre-emulsification was performed manually by the increased number of persons, as shown in Table 3, the rate of the w/o emulsion obtained was found to be 74%. When pre-emulsification was performed with the use of a machine, in contrast, the rate of the w/o emulsion obtained was found to be 63%. The reason why the w/o emulsion could not be obtained at a certain rate when the number of persons and the number of instances to prepare emulsions were increased is considered as follows. That is, vigorous shaking of syringes at the time of pre-emulsification is to be terminated when "a homogeneous white turbid liquid is obtained," and the decision is to be made based on the subjective view of a person who had prepared the emulsion. Thus, it is considered that the resulting emulsion varies depending on a method or force of shaking the syringes by the person who had prepared the emulsion.

**[Table 3]**

| | Number of operation (times) | Frequency of w/o emulsion obtained (times) | Rate of w/o emulsion obtained (%) |
|---|---|---|---|
| Manual pre-emulsification | 19 | 14 | 74 |
| Automated pre-emulsification | 43 | 27 | 63 |

### [Example 3: Preparation of an emulsion using a three-way stopcock]

As described in Example 1, it was found preferable that pre-emulsification be performed under the oil-rich conditions. According to a standard method of emulsion preparation or a simple method of divisional preparation, pre-emulsification can be performed only by shaking syringes even under the oil-rich conditions. Thus, the results are easily affected by a method or force of shaking as described above. When pre-emulsification was performed automatically with the use of a machine in the method of divisional preparation, the rate of the w/o emulsion obtained was approximately 60%. This indicates that a further improvement is desired.

In order to solve the problems described above and increase the rate of the w/o emulsion obtained, a method of emulsion preparation involving the use of a three-way stopcock was designed.

For the method of emulsion preparation involving the use of a three-way stopcock, an empty first syringe 1, a second syringe 2 filled with 1 ml of Montanide ISA 51 VG, and a third syringe 3 filled with 1 ml of a solution of peptides having 4 linked CTL epitopes were prepared (Injekt Syringes, 2 ml, Luer Lock, B. Braun). An apparatus used comprises the empty first syringe 1 connected in a horizontal direction to a three-way stopcock 4 (TS-TR2K, Terumo Corporation), the second syringe 2 filled with Montanide ISA 51 VG directly connected in a horizontal direction to the three-way stopcock 4 via a syringe connector 5 (GP syringe connector, Nipro Corporation) to allow Montanide ISA 51 VG to migrate from the second syringe 2 to the first syringe 1, and the third syringe 3 filled with a solution of peptides having 4 linked CTL epitopes connected in a vertical direction to the three-way stopcock 4, as shown in Fig. 1.

In pre-emulsification, a cock of the three-way stopcock 4 was operated in the manner shown in Fig. 2, so as to allow a liquid to migrate between the first syringe 1 and the third syringe 3, and 0.2 ml of a solution of peptides having 4 linked CTL epitopes filled in the third syringe 3 was injected into the first syringe 1 filled with Montanide ISA 51 VG.

Subsequently, the cock was operated to realize the conditions shown in Fig. 1, so as to allow a liquid to migrate between the first syringe 1 and the second syringe 2, a plunger of the first syringe 1 and that of the second syringe 2 were alternately pushed into the relevant syringe, and a mixture of 0.2 ml of a solution of peptides having 4 linked CTL epitopes and 1 ml of Montanide ISA 51 VG was allowed to reciprocate in horizontal directions between the first syringe 1 and the second syringe 2 via the syringe connector 5 (pre-emulsification). In pre-emulsification, the mixture was allowed to reciprocate between syringes 20 times.

The operation described above was repeated 3 more times by injecting 0.2 ml each of a solution of peptides having 4 linked CTL epitopes into the first syringe 1 from the third syringe 3. The solution of peptides having 4 linked CTL epitopes (0.2 ml each) was injected into the first syringe 1 from the third syringe 3, this operation was repeated 5 times, and the mixture of the solution of peptides having 4 linked CTL epitopes and 1 ml of Montanide ISA 51 VG was allowed to reciprocate in horizontal directions between the first syringe 1 and the second syringe 2 via the syringe connector 5, 60 times (main emulsification). Whether or not a w/o emulsion was prepared was determined by a drop test. The resulting emulsion was confirmed to be a w/o emulsion by the drop test.

### [Example 4: Examination of the number of reciprocation between syringes in pre-emulsification]

In order to further optimize a method for preparing an emulsion using a three-way stopcock, the number of mixing to be performed in pre-emulsification was examined. The method for preparing an emulsion using a three-way stopcock was performed using the apparatus as used in Example 3 in the same manner as in Example 3, except for the number of reciprocation between syringes at the time of pre-emulsification.

In the method described above, an emulsion was prepared by allowing the mixture to reciprocate between syringes 10, 15, or 20 times in a single operation of pre-emulsification.

When the mixture was allowed to reciprocate between syringes 10 to 20 times at the time of pre-emulsification, the emulsion was not easily dispersed in water, and a w/o emulsion was obtained. The results demonstrate that operation efficiency can be improved by allowing the mixture to reciprocate between syringes using a three-way stopcock at the time of pre-emulsification, compared with pre-emulsification performed by vigorously shaking syringes in a standard method of emulsion preparation.

### [Example 5: Examination of the number of reciprocation between syringes in main emulsification]

The number of reciprocation between syringes to be repeated in main emulsification after the pre-emulsification as performed in Example 4, in which the mixture was allowed to reciprocate 10 times, was examined. Specifically, the mixture was allowed to reciprocate between syringes 10, 20, or 30 times in main emulsification and examined. For comparison, the number of reciprocation between syringes to be repeated in main emulsification in the method of divisional preparation (the number of divisional mixing in pre-emulsification performed in Example 1: 5) was examined.

Whether or not a w/o emulsion could be prepared was determined by performing a drop test. Particle sizes of the emulsions prepared via mixing a given number of times were measured using a wet/dry particle size distribution analyzer LS 13 320 (Beckman Coulter) (the laser diffraction scattering method).

The conditions for measurement are shown below.
Module used: Universal liquid module (wet particle size diameter measurement)
Dispersion medium: Mineral oil, light white
Refractive index of a dispersion medium: 1.45
Ultrasonic dispersion: none
Method for preparing a sample: A drop of an emulsion was primarily dispersed in 10 ml of the mineral oil, light white.

According to the method of emulsion preparation using a three-way stopcock, a w/o emulsion was obtained without being easily dispersed in water, regardless of the number of reciprocation between syringes at the time of main emulsification. According to the method of divisional preparation, a drop test was performed only when the mixture was allowed to reciprocate between syringes 60 times in main emulsification, and it was confirmed that a w/o emulsion was obtained.

As shown in Fig. 3, variation in particle sizes of the resulting emulsions was observed when the number of reciprocation between syringes was 20 or less in main emulsification according to the method of divisional preparation. In contrast, it was found that uniform and approximately the same particle size could be attained when the mixture was allowed to reciprocate between syringes 30 times or more. According to the method of emulsion preparation using a three-way stopcock, as shown in Fig. 4, uniform and approximately the same particle size was attained when the number of reciprocation was 10 or more.

### [Example 6: Examination of reproducibility in the method of emulsion preparation using a three-way stopcock]

According to the method of emulsion preparation using a three-way stopcock in Example 5, an emulsion was prepared under the conditions in which the number of reciprocation between syringes was designated 20 at the time of main emulsification, the particle size and the viscosity of the resulting emulsion were measured, and reproducibility among preparation instances was examined.

The particle size was measured in the same manner as in Example 5 and the viscosity was measured under the conditions described below.
Apparatus used: type E viscometer (TOKYO KEIKI In.)
Cone: 1°34'
Sample amount: approximately 1 ml
Measurement temperature: 30°C
Number of revolution: 10 rpm

As shown in Fig. 5, particle sizes of the emulsions obtained by 3 preparation instances were uniform and approximately the same, and the particle sizes were within the range of 1 to 3 µm. As shown in Table 4, in addition, no significant variation was observed in the viscosity of the emulsions obtained by 3 preparation instances.

**[Table 4]**

| | 1st | 2nd | 3rd | Average | S.D. |
|---|---|---|---|---|---|
| Viscosity (mPa·s) | 305.4 | 276.2 | 357.1 | 312.9 | 41.0 |

### [Example 7: Method of emulsion preparation using a three-way stopcock]

Under the conditions of Example 6, the number of persons and the number of instances for preparing emulsions were increased, and the rate of the w/o emulsions obtained was examined. As shown in Table 5, the rate of the w/o emulsions obtained was found to be 98%.

**[Table 5]**

| Number of operation (times) | Frequency of w/o emulsion obtained (times) | Rate of w/o emulsion obtained (%) |
|---|---|---|
| 52 | 51 | 98 |

The above results demonstrate that it was very difficult to obtain the w/o emulsion formulation comprising 3 types of peptides having 4 linked CTL epitopes of the present invention by a standard method of emulsion preparation. In contrast, the w/o emulsion was obtained when a solution of peptides having 4 linked CTL epitopes (an aqueous phase) was divided into sections and the sections were divisionally injected into an oil phase comprising an oil adjuvant when mixing the aqueous phase with the oil phase (i.e., the method of divisional preparation). According to the method of emulsion preparation using a three-way stopcock, in addition, the w/o emulsion was obtained at a higher rate. Compared with a standard method of emulsion preparation or a standard method of divisional preparation, further, the w/o emulsion could be obtained with less effort in a shorter period of time.

### [Description of numeral references]

- 1:: First syringe
- 2:: Second syringe
- 3:: Third syringe
- 4:: Three-way stopcock
- 5:: Syringe connector
- 6:: Syringe connector tip

All publications, patents, and patent applications cited herein are incorporated herein by reference in their entirety.

## Claims

1. A water-in-oil (w/o) emulsion formulation containing 3 types of peptides having 4 linked CTL epitopes each comprising 4 CTL epitope peptides linked via linkers in an aqueous phase.

2. The water-in-oil emulsion formulation according to claim 1, which contains 3 types of peptides having 4 linked CTL epitopes and an oil adjuvant.

3. The water-in-oil emulsion formulation according to claim 1 or 2, which is obtained by mixing an aqueous phase comprising peptides having 4 linked CTL epitopes and an oil phase comprising an oil adjuvant and emulsifying the mixture, wherein the aqueous phase comprising peptides having 4 linked CTL epitopes is a solution comprising 3 types of peptides having 4 linked CTL epitopes: a peptide represented by Formula (I); a peptide represented by Formula (II); and a peptide represented by Formula (III):
A-(L)-B-(L)-C-(L)-PEP2 (I);
D-(L)-E-(L)-F-(L)-PEP4 (II); and
G-(L)-H-(L)-I-(L)-PEP10 (III),
in Formulae (I), (II), and (III),
(L) represents a linker;
PEP2 represents a CTL epitope peptide represented by SEQ ID NO: 2;
PEP4 represents a CTL epitope peptide represented by SEQ ID NO: 4;
PEP10 represents a CTL epitope peptide represented by SEQ ID NO: 10;
A, B, C, D, E, F, G, H, and I are each independently selected from the group consisting of a CTL epitope peptide represented by SEQ ID NO: 1 (PEP1), a CTL epitope peptide represented by SEQ ID NO: 5 (PEP5), a CTL epitope peptide represented by SEQ ID NO: 6 (PEP6), a CTL epitope peptide represented by SEQ ID NO: 7 (PEP7), a CTL epitope peptide represented by SEQ ID NO: 8 (PEP8), a CTL epitope peptide represented by SEQ ID NO: 9 (PEP9), a CTL epitope peptide represented by SEQ ID NO: 13 (PEP13), a CTL epitope peptide represented by SEQ ID NO: 15 (PEP15), and a CTL epitope peptide represented by SEQ ID NO: 18 (PEP18), except for a combination of A representing PEP7 and B representing PEP8; and
the peptides having 4 linked CTL epitopes represented by Formulae (I), (II), and (III) may each have a peptide sequence consisting of hydrophilic amino acids.

4. The emulsion formulation according to claim 3, wherein, in Formulae (I), (II), and (III), (L) represents a linker; A, B, and C are each independently selected from the group consisting of PEP7, PEP8, and PEP13, except for a combination of A representing PEP7 and B representing PEP8; D, E, and F are each independently selected from the group consisting of PEP5, PEP6, and PEP9; and G, H, and I are each independently selected from the group consisting of PEP1, PEP15, and PEP18, and the peptide represented by Formula (I) may have a peptide sequence consisting of hydrophilic amino acids.

5. The emulsion formulation according to claim 3 or 4, wherein the peptide represented by Formula (I) is selected from the group consisting of:
▪ PEP7-(L)-PEP13-(L)-PEP8-(L)-PEP2;
▪ PEP8-(L)-PEP7-(L)-PEP13-(L)-PEP2;
▪ PEP8-(L)-PEP13-(L)-PEP7-(L)-PEP2;
▪ PEP13-(L)-PEP7-(L)-PEP8-(L)-PEP2; and
▪ PEP13-(L)-PEP8-(L)-PEP7-(L)-PEP2,
the peptide represented by Formula (II) is selected from the group consisting of:
▪ PEP5-(L)-PEP6-(L)-PEP9-(L)-PEP4;
▪ PEP5-(L)-PEP9-(L)-PEP6-(L)-PEP4;
▪ PEP6-(L)-PEP5-(L)-PEP9-(L)-PEP4;
▪ PEP6-(L)-PEP9-(L)-PEP5-(L)-PEP4;
▪ PEP9-(L)-PEP5-(L)-PEP6-(L)-PEP4; and
▪ PEP9-(L)-PEP6-(L)-PEP5-(L)-PEP4, and
the peptide represented by Formula (III) is selected from the group consisting of:
▪ PEP1-(L)-PEP15-(L)-PEP18-(L)-PEP10;
▪ PEP1-(L)-PEP18-(L)-PEP15-(L)-PEP10;
▪ PEP15-(L)-PEP1-(L)-PEP18-(L)-PEP10;
▪ PEP15-(L)-PEP18-(L)-PEP1-(L)-PEP10;
▪ PEP18-(L)-PEP1-(L)-PEP15-(L)-PEP10; and
▪ PEP18-(L)-PEP15-(L)-PEP1-(L)-PEP10, and
the peptide represented by Formula (I) may have a peptide sequence consisting of hydrophilic amino acids at the N terminus.

6. The emulsion formulation according to any one of claims 3 to 5, wherein, in Formulae (I), (II), and (III), (L) represents an arginine dimer; and a peptide sequence consisting of hydrophilic amino acids is an arginine tetramer.

7. The emulsion formulation according to any one of claims 3 to 6, wherein the 3 types of peptides having 4 linked CTL epitopes represented by Formulae (I), (II), and (III) are RRRR-PEP7-RR-PEP13-RR-PEP8-RR-PEP2 (SEQ ID NO: 19), PEP5-RR-PEP9-RR-PEP6-RR-PEP4 (SEQ ID NO: 25), and PEP15-RR-PEP18-RR-PEP1-RR-PEP10 (SEQ ID NO: 33).

8. A method for preparing the emulsion formulation according to any one of claims 1 to 7 comprising the steps (i) and (ii):
(i) a step of injecting a part of an aqueous phase comprising peptides having 4 linked CTL epitopes into an oil phase comprising an oil adjuvant to prepare a mixture; and
(ii) a step of allowing the mixture to reciprocate between syringes connected via a syringe connector,
wherein the step (i) and the step (ii) are repeated to obtain an emulsion formulation comprising the total amount of the oil phase and the aqueous phase.

9. The method according to claim 8, wherein the amount of a part of the aqueous phase is 1/10 to 1/2 fold (v/v) relative to the amount of the oil phase comprising an oil adjuvant.

10. An apparatus used for preparing a water-in-oil emulsion formulation containing peptides, which is composed of:
a second syringe 2;
a syringe connector 5 connected to the second syringe 2;
a three-way stopcock 4 connected to the syringe connector 5; and
a first syringe 1 and a third syringe 3 further connected to the three-way stopcock 4,
wherein the apparatus enables production of a water-in-oil emulsion formulation by adding an aqueous phase filled in the third syringe 3 to the oil phase filled in the first syringe 1 or the second syringe 2 and allowing the resulting mixture to reciprocate between the first syringe 1 and the second syringe 2 via the syringe connector 5.

11. The apparatus according to claim 10, wherein the volumes of the first, the second, and the third syringes are each 3 ml or less.

12. The apparatus according to claim 10 or 11, wherein an inner diameter of the syringe connector 5 through which the mixture passes is within a range from 0.8 to 1.7 mm.

13. A kit used for preparing an w/o emulsion containing peptides having 4 linked CTL epitopes, which comprises peptides having 4 linked CTL epitopes each comprising 4 CTL epitope peptides linked via linkers, an oil adjuvant, and the apparatus according to any one of claims 10 to 12.

14. A method for treatment of tumor of a patient comprising administering the water-in-oil (w/o) emulsion formulation according to any one of claims 1 to 7 to a patient.

15. The water-in-oil (w/o) emulsion formulation according to any one of claims 1 to 7 for use in the treatment of tumor.

16. Use of a solution of peptides having 4 linked CTL epitopes in the manufacture of the water-in-oil (w/o) emulsion formulation according to any one of claims 1 to 7.

17. The apparatus according to any one of claims 10 to 12 or the kit according to claim 13, which is used for preparing the water-in-oil (w/o) emulsion formulation for the treatment of tumor at the time of use.

18. The apparatus according to any one of claims 10 to 12 or the kit according to claim 13, wherein the water-in-oil (w/o) emulsion formulation is the formulation according to any one of claims 1 to 7.

19. A method for treatment of tumor of a patient comprising:
(a) preparing the emulsion formulation according to any one of claims 1 to 7 by performing the step (i) and the step (ii):
(i) a step of injecting a part of an aqueous phase comprising peptides having 4 linked CTL epitopes into an oil phase comprising an oil adjuvant to prepare a mixture; and
(ii) a step of allowing the mixture to reciprocate between syringes connected via a syringe connector, and
repeating the step (i) and the step (ii) to obtain the emulsion formulation comprising the total amount of the oil phase and the aqueous phase; and
(b) administering the resulting emulsion formulation to a patient having tumor.


SEQUENCE LISTING

<110>  TAIHO PHARMACEUTICAL CO., LTD.

<120>  Emulsion Formulation and Preparation Method thereof

<130>  PH-8112-PCT

<150>  JP 2018-207779
<151>  2018-11-02

<160>  35

<170>  PatentIn version 3.5

<210>  1
<211>  9
<212>  PRT
<213>  Homo sapiens

<400>  1

Lys Leu Val Glu Arg Leu Gly Ala Ala
1               5


<210>  2
<211>  9
<212>  PRT
<213>  Homo sapiens

<400>  2

Ala Ser Leu Asp Ser Asp Pro Trp Val
1               5


<210>  3
<211>  9
<212>  PRT
<213>  Homo sapiens

<400>  3

Ala Leu Val Glu Phe Glu Asp Val Leu
1               5


<210>  4
<211>  9
<212>  PRT
<213>  Homo sapiens

<400>  4

Leu Leu Gln Ala Glu Ala Pro Arg Leu
1               5


<210>  5
<211>  9
<212>  PRT
<213>  Homo sapiens

<400>  5

Asp Tyr Ser Ala Arg Trp Asn Glu Ile
1               5


<210>  6
<211>  10
<212>  PRT
<213>  Homo sapiens

<400>  6

Val Tyr Asp Tyr Asn Cys His Val Asp Leu
1               5                   10


<210>  7
<211>  9
<212>  PRT
<213>  Homo sapiens

<400>  7

Leu Tyr Ala Trp Glu Pro Ser Phe Leu
1               5


<210>  8
<211>  10
<212>  PRT
<213>  Homo sapiens

<400>  8

Asp Tyr Leu Arg Ser Val Leu Glu Asp Phe
1               5                   10


<210>  9
<211>  9
<212>  PRT
<213>  Homo sapiens

<400>  9

Gln Ile Arg Pro Ile Phe Ser Asn Arg
1               5


<210>  10
<211>  10
<212>  PRT
<213>  Homo sapiens

<400>  10

Ile Leu Glu Gln Ser Gly Glu Trp Trp Lys
1               5                   10


<210>  11

<211>  10
<212>  PRT
<213>  Homo sapiens

<400>  11

Val Ile Gln Asn Leu Glu Arg Gly Tyr Arg
1               5                   10


<210>  12
<211>  10
<212>  PRT
<213>  Homo sapiens

<400>  12

Lys Leu Lys His Tyr Gly Pro Gly Trp Val
1               5                   10


<210>  13
<211>  9
<212>  PRT
<213>  Homo sapiens

<400>  13

Arg Leu Gln Glu Trp Cys Ser Val Ile
1               5


<210>  14
<211>  9
<212>  PRT
<213>  Homo sapiens

<400>  14

Ile Leu Gly Glu Leu Arg Glu Lys Val
1               5


<210>  15
<211>  10
<212>  PRT
<213>  Homo sapiens

<400>  15

Asp Tyr Val Arg Glu His Lys Asp Asn Ile
1               5                   10


<210>  16
<211>  9
<212>  PRT
<213>  Homo sapiens

<400>  16

His Tyr Thr Asn Ala Ser Asp Gly Leu
1               5

<210>  17
<211>  10
<212>  PRT
<213>  Homo sapiens

<400>  17

Asn Tyr Ser Val Arg Tyr Arg Pro Gly Leu
1               5                   10


<210>  18
<211>  10
<212>  PRT
<213>  Homo sapiens

<400>  18

Arg Tyr Leu Thr Gln Glu Thr Asn Lys Val
1               5                   10


<210>  19
<211>  47
<212>  PRT
<213>  Artificial

<220>
<223>  Synthetic peptide

<400>  19

Arg Arg Arg Arg Leu Tyr Ala Trp Glu Pro Ser Phe Leu Arg Arg Arg
1               5                   10                  15


Leu Gln Glu Trp Cys Ser Val Ile Arg Arg Asp Tyr Leu Arg Ser Val
            20                  25                  30


Leu Glu Asp Phe Arg Arg Ala Ser Leu Asp Ser Asp Pro Trp Val
        35                  40                  45


<210>  20
<211>  47
<212>  PRT
<213>  Artificial

<220>
<223>  Synthetic peptide

<400>  20

Arg Arg Arg Arg Asp Tyr Leu Arg Ser Val Leu Glu Asp Phe Arg Arg
1               5                   10                  15


Leu Tyr Ala Trp Glu Pro Ser Phe Leu Arg Arg Arg Leu Gln Glu Trp
            20                  25                  30

Cys Ser Val Ile Arg Arg Ala Ser Leu Asp Ser Asp Pro Trp Val
        35                  40                  45

<210>  21
<211>  47
<212>  PRT
<213>  Artificial

<220>
<223>  Synthetic peptide

<400>  21

Arg Arg Arg Arg Asp Tyr Leu Arg Ser Val Leu Glu Asp Phe Arg Arg
1               5                   10                  15

Arg Leu Gln Glu Trp Cys Ser Val Ile Arg Arg Leu Tyr Ala Trp Glu
            20                  25                  30

Pro Ser Phe Leu Arg Arg Ala Ser Leu Asp Ser Asp Pro Trp Val
        35                  40                  45

<210>  22
<211>  47
<212>  PRT
<213>  Artificial

<220>
<223>  Synthetic peptide

<400>  22

Arg Arg Arg Arg Arg Leu Gln Glu Trp Cys Ser Val Ile Arg Arg Leu
1               5                   10                  15

Tyr Ala Trp Glu Pro Ser Phe Leu Arg Arg Asp Tyr Leu Arg Ser Val
            20                  25                  30

Leu Glu Asp Phe Arg Arg Ala Ser Leu Asp Ser Asp Pro Trp Val
        35                  40                  45

<210>  23
<211>  47
<212>  PRT
<213>  Artificial

<220>
<223>  Synthetic peptide

<400>  23

Arg Arg Arg Arg Arg Leu Gln Glu Trp Cys Ser Val Ile Arg Arg Asp
1               5                   10                  15

Tyr Leu Arg Ser Val Leu Glu Asp Phe Arg Arg Leu Tyr Ala Trp Glu
            20                  25                  30

Pro Ser Phe Leu Arg Arg Ala Ser Leu Asp Ser Asp Pro Trp Val
        35                  40                  45

<210>  24
<211>  43
<212>  PRT
<213>  Artificial

<220>
<223>  Synthetic peptide

<400>  24

Asp Tyr Ser Ala Arg Trp Asn Glu Ile Arg Arg Val Tyr Asp Tyr Asn
1               5                   10                  15

Cys His Val Asp Leu Arg Arg Gln Ile Arg Pro Ile Phe Ser Asn Arg
            20                  25                  30

Arg Arg Leu Leu Gln Ala Glu Ala Pro Arg Leu
        35                  40

<210>  25
<211>  43
<212>  PRT
<213>  Artificial

<220>
<223>  Synthetic peptide

<400>  25

Asp Tyr Ser Ala Arg Trp Asn Glu Ile Arg Arg Gln Ile Arg Pro Ile
1               5                   10                  15

Phe Ser Asn Arg Arg Arg Val Tyr Asp Tyr Asn Cys His Val Asp Leu
            20                  25                  30

Arg Arg Leu Leu Gln Ala Glu Ala Pro Arg Leu
        35                  40

<210>  26
<211>  43
<212>  PRT
<213>  Artificial

<220>
<223>  Synthetic peptide

<400>  26

Val Tyr Asp Tyr Asn Cys His Val Asp Leu Arg Arg Asp Tyr Ser Ala

1               5                   10                  15

Arg Trp Asn Glu Ile Arg Arg Gln Ile Arg Pro Ile Phe Ser Asn Arg
            20                  25                  30

Arg Arg Leu Leu Gln Ala Glu Ala Pro Arg Leu
        35                  40

<210>  27
<211>  43
<212>  PRT
<213>  Artificial

<220>
<223>  Synthetic peptide

<400>  27

Val Tyr Asp Tyr Asn Cys His Val Asp Leu Arg Arg Gln Ile Arg Pro
1               5                   10                  15

Ile Phe Ser Asn Arg Arg Arg Asp Tyr Ser Ala Arg Trp Asn Glu Ile
            20                  25                  30

Arg Arg Leu Leu Gln Ala Glu Ala Pro Arg Leu
        35                  40

<210>  28
<211>  43
<212>  PRT
<213>  Artificial

<220>
<223>  Synthetic peptide

<400>  28

Gln Ile Arg Pro Ile Phe Ser Asn Arg Arg Arg Asp Tyr Ser Ala Arg
1               5                   10                  15

Trp Asn Glu Ile Arg Arg Val Tyr Asp Tyr Asn Cys His Val Asp Leu
            20                  25                  30

Arg Arg Leu Leu Gln Ala Glu Ala Pro Arg Leu
        35                  40

<210>  29
<211>  43
<212>  PRT
<213>  Artificial

<220>
<223>  Synthetic peptide

<400>  29

Gln Ile Arg Pro Ile Phe Ser Asn Arg Arg Arg Val Tyr Asp Tyr Asn
1               5                   10                  15

Cys His Val Asp Leu Arg Arg Asp Tyr Ser Ala Arg Trp Asn Glu Ile
            20                  25                  30

Arg Arg Leu Leu Gln Ala Glu Ala Pro Arg Leu
        35                  40

<210>  30
<211>  45
<212>  PRT
<213>  Artificial

<220>
<223>  Synthetic peptide

<400>  30

Lys Leu Val Glu Arg Leu Gly Ala Ala Arg Arg Asp Tyr Val Arg Glu
1               5                   10                  15

His Lys Asp Asn Ile Arg Arg Arg Tyr Leu Thr Gln Glu Thr Asn Lys
            20                  25                  30

Val Arg Arg Ile Leu Glu Gln Ser Gly Glu Trp Trp Lys
        35                  40                  45

<210>  31
<211>  45
<212>  PRT
<213>  Artificial

<220>
<223>  Synthetic peptide

<400>  31

Lys Leu Val Glu Arg Leu Gly Ala Ala Arg Arg Arg Tyr Leu Thr Gln
1               5                   10                  15

Glu Thr Asn Lys Val Arg Arg Asp Tyr Val Arg Glu His Lys Asp Asn
            20                  25                  30

Ile Arg Arg Ile Leu Glu Gln Ser Gly Glu Trp Trp Lys
        35                  40                  45

<210>  32
<211>  45
<212>  PRT
<213>  Artificial

<220>
<223>  Synthetic peptide

<400>  32

Asp Tyr Val Arg Glu His Lys Asp Asn Ile Arg Arg Lys Leu Val Glu
1               5                   10                  15

Arg Leu Gly Ala Ala Arg Arg Arg Tyr Leu Thr Gln Glu Thr Asn Lys
            20                  25                  30

Val Arg Arg Ile Leu Glu Gln Ser Gly Glu Trp Trp Lys
        35                  40                  45

<210>  33
<211>  45
<212>  PRT
<213>  Artificial

<220>
<223>  Synthetic peptide

<400>  33

Asp Tyr Val Arg Glu His Lys Asp Asn Ile Arg Arg Arg Tyr Leu Thr
1               5                   10                  15

Gln Glu Thr Asn Lys Val Arg Arg Lys Leu Val Glu Arg Leu Gly Ala
            20                  25                  30

Ala Arg Arg Ile Leu Glu Gln Ser Gly Glu Trp Trp Lys
        35                  40                  45

<210>  34
<211>  45
<212>  PRT
<213>  Artificial

<220>
<223>  Synthetic peptide

<400>  34

Arg Tyr Leu Thr Gln Glu Thr Asn Lys Val Arg Arg Lys Leu Val Glu
1               5                   10                  15

Arg Leu Gly Ala Ala Arg Arg Asp Tyr Val Arg Glu His Lys Asp Asn
            20                  25                  30

Ile Arg Arg Ile Leu Glu Gln Ser Gly Glu Trp Trp Lys
        35                  40                  45

<210>  35
<211>  45

<212>  PRT
<213>  Artificial

<220>
<223>  Synthetic peptide

<400>  35

Arg Tyr Leu Thr Gln Glu Thr Asn Lys Val Arg Arg Asp Tyr Val Arg
1               5                   10                  15

Glu His Lys Asp Asn Ile Arg Arg Lys Leu Val Glu Arg Leu Gly Ala
            20                  25                  30

Ala Arg Arg Ile Leu Glu Gln Ser Gly Glu Trp Trp Lys
        35                  40                  45